(19) Europäisches Patentamt
European Patent Office
Office européen des brevets

(11) EP 0 799 836 A1

(12) **EUROPEAN PATENT APPLICATION**
published in accordance with Art. 158(3) EPC

(43) Date of publication:
**08.10.1997 Bulletin 1997/41**

(21) Application number: **95942277.5**

(22) Date of filing: **27.12.1995**

(51) Int. Cl.[6]: **C07K 16/40**, C07K 16/18, C12P 21/08, C12N 15/06, C12N 15/13, C12N 5/20, C12N 9/99, A61K 39/395 // (C12P21/08, C12R1:91)

(86) International application number:
**PCT/JP95/02714**

(87) International publication number:
**WO 96/20959 (11.07.1996 Gazette 1996/31)**

(84) Designated Contracting States:
**AT BE CH DE DK ES FR GB GR IE IT LI LU NL PT SE**

(30) Priority: **29.12.1994 JP 340006/94**

(71) Applicant:
**YAMANOUCHI PHARMACEUTICAL CO. LTD.**
**Tokyo 103 (JP)**

(72) Inventors:
- **KAWAUCHI, Yasushi**
  **Ibaraki 300 (JP)**
- **TAKASAKI, Jun**
  **Ibaraki 300 (JP)**
- **YASUNAGA, Tomoe**
  **Ibaraki 305 (JP)**
- **MASUHO, Yasuhiko**
  **Tokyo 184 (JP)**

(74) Representative: **Kolb, Helga, Dr. Dipl.-Chem. et al**
**Hoffmann Eitle,**
**Patent- und Rechtsanwälte,**
**Arabellastrasse 4**
**81925 München (DE)**

# (54) NOVEL MONOCLONAL ANTIBODY HAVING INHIBITORY EFFECT ON TYPE II PHOSPHOLIPASE A2 AND PROTEIN CONTAINING A PART OF THE SAME

(57) A novel monoclonal antibody capable of inhibiting the activity of type II phospholipase A2 was obtained. Since said antibody is able to inhibit the activity of human type II phospholipase A2 as well as monkey type II phospholipase A2 and/or mouse type II phospholipase A2, it is not only clinically useful, but also usable as such in preclinical tests with the use of monkey or mouse. The antibody, which a completely novel property of releasing type II phospholipase A2 bound to the cell membrane, seems to strongly inhibit the activity of said enzyme via a novel mechanism. The monoclonal antibody or protein comprising a part thereof of the present invention can be used as therapeutics for cardiac infarction, cerebral infarction, etc. wherein type II phospholipase A2 is involved.

EP 0 799 836 A1

## Description

### Field of the Invention

The present invention relates to novel monoclonal antibodies having potent inhibitory activity against type II phospholipase A2, and proteins comprising a part thereof.

More specifically, the present invention relates to novel monoclonal antibodies which are superior in the specificity and affinity for type II phospholipase A2, highly inhibitory against type II phospholipase A2, and usable as therapeutics for the treatment of diseases related with type II phospholipase A2 and protein comprising a part thereof. Furthermore, the present invention relates to cells producing said monoclonal antibodies or proteins, DNAs encoding said monoclonal antibodies or proteins, and recombinant vectors comprising said DNAs. In addition, the present invention relates to medical compositions comprising said monoclonal antibodies or proteins, or inhibitor of said type II phospholipase A2.

### Background of the Invention

Phospholipase A2 is known as the enzyme to catalyze the hydrolysis of the ester bond in position C2 of 1,2-acylphosphoglyceride, a component of biomembrane. This enzyme is distributed in various organs and cells of mammals, and not only regulates the generation and metabolism of phospholipid of biomembrane, but also acts as the rate-limiting enzyme in the arachidonic acid cascade, the metabolic products of which including prostaglandin, leukotriene, thromboxane, PAF etc. are known to have a variety of physiological activities.

Several types of phospholipase A2, such as type I, type II, intracellular type, etc. are known (Atsumi I. et al., Nippon Rinsho, Extra edition 1994, 202-206). Among them, type II phospholipase A2 is induced in the exudate of inflammatory site, and released in large quantities in a blood stream during the inflammatory reaction. Several reports indicate that this enzyme exacerbates the symptom of various inflammatory diseases or is a part of its cause. For example, Kikuchi-Yanoshita et al. reported the elevation of this enzyme activity in rat with ischemic cardiopathy as an animal model of cardiac infarction when the organ impairment is advanced (Kikuchi-Yanoshita R. et al.,J.Biochemistry, 114, 33-38, 1993). Leong et al. reported the presence of a larger quantity of this enzyme in a blood stream of patients with cardiac infarction than normal subjects (Leong L. L. et al., Clinical Experimental Pharmacology and Physiology, 19: 113-118, 1992).

Lauritzen et al. reported to indicate that this enzyme is playing an important role in the exacerbation of symptoms of the ischemic reperfusion disorder in patients with cerebral infarction (Lauritzen I. et al., Brain Research, 651: 353-356, 1994). Bauer et al. also reported that this enzyme plays an important role in exacerbating symptoms in the acute renal insufficiency (Bauer M., Klin. Wochenschr., 67: 196-202, 1989). Murakami et al. suggested that this enzyme stimulates the histamine release from mast cells which play central roles in allergic diseases such as asthma, etc. and also that the inhibition of this enzyme may lead to a possible development of therapeutics for the treatment of allergic diseases including asthma, etc. (Murakami M. et al., Journal of Immunology, 151: 5675-5684, 1993).

Smith et al. and Pruzanski et al. reported the presence of larger quantities of this enzyme in a blood stream of patients with chronic rheumatoid arthritis than normal subjects, suggesting a possibility that this enzyme causes or exacerbates the disease (Smith G. M. et al., British Journal of Rheumatology, 31: 175-178, 1992; Pruzanski W. et al., J. Rheumatol., 15: 1351-1355, 1988). Pruzanski et al. similarly reported the presence of larger quantities of this enzyme in exudates of patients with osteoarthritis than normal subjects, suggesting a possibility of this enzyme to be a cause of the disease or its exacerbation (Pruzanski W. et al., Life Sciences, 48: 2457-2462, 1991). Vadas et al. and Green et al. reported the presence of larger quantities of this enzyme in a blood stream of patient suffering from septic shock than normal subject, suggesting this enzyme being a possible cause of the disease or its exacerbation (Vadas P. et al., Life Sciences, 50: 807-811, 1992; Green, J., Inflammation, 15: 355-367, 1991).

Nevalainen et al. reported the presence of large quantities of this enzyme in a blood stream of patients with especially severe pancreatitis, suggesting a possibility of this enzyme being a cause or of the disease or its exacerbation (Nevalainen T. J. et al., Gut, 34, 1133-1136, 1993). Anderson et al. reported the presence of said enzyme in large quantities in the skin of patients with psoriasis, suggesting a possibility of this enzyme being a cause of the disease or its exacerbation (Anderson S. et al., Inflammation, 18: 1-12, 1994).

Koike et al. reported to suggest that this enzyme plays a main role as the cause of multiple organ failure (MOF) (Koike K. et al. Surgery, 112: 173-180, 1992). Koeniger et al., Edelson et al. and Romaschin et al. reported the presence of large quantities of this enzyme in the blood stream of patients with acute respiratory distress syndrome (ARDS) than normal subjects, suggesting a possibility of this enzyme being a cause of the disease or its exacerbation (Koeniger R. et al., Klin. Wochenschr., 67: 212-216, 1989; Edelson J. D., et al., Am. Rev. Respir. Dis., 143: 1102-1109, 1991; Romaschin A. et al., Clin. Biochem., 25: 55-60, 1992).

Minami et al. reported the presence of large quantities of this enzyme in the blood stream of patients with Crohn's disease and ulcerative colitis, suggesting a possibility of this enzyme being the cause of the diseases or their exacerbation (Minaki T. et al., Gut, 33: 914-921, 1992).

In addition, this enzyme has been indicated to be involved with uveitis, respiratory distress syndrome of the newborn (RDS), bronchopulmonary dysplasia (BRD), etc.

As the anti-human type II phospholipase A2 antibody, the following antibodies are reported.

Stoner et al. purified human type II phospholipase A2 from human placenta and synovial fluid, and, using the purified enzyme as antigen, obtained a monoclonal antibody raised against it in mouse (Stoner C. R. et al., Journal of Immunological Methods, 145: 127-136, 1991). In this method, they first sensitized mouse of BALB/cByJ strain with the purified phospholipase A2 mixed with Freund's complete adjuvant (5 μg), followed by the same enzyme (3μg) 25 days later. Furthermore, they sensitized the mouse with the same preparation (5 μg) 3 days prior to fusion of spleen cells (36 days after the initial sensitization). Therefore, they immunized the mouse with the immunogen totaling 13 μg, divided in three portions over a period of 36 days. Spleen cells isolated were fused with mouse myeloma PAI-0 cells, and screened in HAT medium. Supernatant of the medium was examined by ELISA system using phospholipase A2 to obtain the monoclonal antibodies (PLA184, PLA185, PLA186 and PLA187). These antibodies were certainly capable of inhibiting human phospholipase A2. However, the inhibitory activity of these antibodies against phospholipase A2 of other animal species was examined using only rat type II phospholipase A2. They reported that these antibodies showed little or no cross-reaction activity with rat type II phospholipase A2, and, accordingly, could not be used in animal experiments with rats.

Takayama et al. also purified human type II phospholipase A2 from synovia of a patients with rheumatism, and, using this purified enzyme as antigen, obtained a monoclonal antibody in mouse (Takayama K. et al., Biochemical and Biophysical Research Communications, 167: 1309-1315, 1990). In this immunization method, they adsorbed the purified human type II phospholipase A2 (10 μg) on nitrocellulose membrane, homogenized and injected resulting homogenate into the spleen of BALB/c strain mouse, followed by a similar immunization 2 weeks later. Therefore, they immunized the animal with the immunizing antigen of 20 μg in total, divided in two equal portions over a period of 28 days, and then, 3 days later, performed the fusion of spleen cells with mouse myeloma cells (X63-Ag8.6.5.3). They examined the supernatant of the medium by ELISA system using phospholipase A2 to obtain the reactive monoclonal antibodies (HP-1, HP-2, HP-3 and HP-4). Among them, HP-1 showed the highest inhibitory activity to human type II phospholipase A2. However, even HP-1 showed only weak inhibitory activity, inhibiting about 80% even by the addition in its 200 molar excess.

McCord et al. purified a recombinant human type II phospholipase A2, and obtained a monoclonal antibody in mouse using this purified enzyme as antigen (McCord M. et al., The Journal of Investigative Dermatology, 102: 980-986, 1994). For immunizing animals, they immunized mouse of CAF1 strain with phospholipase A2 mixed with Freund's complete adjuvant in amounts of 100, 50 and 25 μg, respectively every four weeks with the final immunization performed 3 days prior to the fusion of spleen cells. Thus, about 200 μg in total of the immunogen divided in four portions were immunized over a period of 56 days. Spleen cells were fused with mouse myeloma cells SP2/0-AG14, and screened for positive cells in HAT medium. Supernatant of the medium was examined for the inhibitory activity using phospholipase A2 to obtain a monoclonal antibody (3F10). They examined the inhibitory activity of this antibody (1 μg) to human phospholipase A2, but without specifying the amount of the enzyme used, making it difficult to estimate its inhibitory potency.

Furthermore, in Tokkai H4-506447, Johnson L. K. disclosed the purification of a recombinant human phospholipase A2 and the preparation of a polyclonal antibody in rabbit using the purified enzyme as immunogen. This antibody is not monoclonal, and, furthermore, he used two kinds of peptide as antigens, which are parts of the human phospholipase A2 sequence, "GTKFLSYKFSNSGSRITC" (amino acids 67-85 from the N terminus) and "NKTTYNKKYQYYSNKHSRGSTPRC" (amino acids 109-132 from the N terminus). He assayed the inhibitory capability of this antibody without specifying amounts of the antibody and human phospholipase A2 used in the specification, making it difficult to estimate its inhibitory potency.

Also, in Tokkai H7-109300, an antibody which stimulates the binding of human phospholipase A2 to sulfated polysaccharide is disclosed without confirming the inhibition of human type II phospholipase A2 by said antibody.

Furthermore, there is no information about the inhibitory activity of said known antibodies discribed above against type II phospholipase A2 from monkey, mouse, rabbit, cat, dog and rat, or their potencies to release human type II phospholipase A2 bound to the cell.

Upon immunizing mouse with the human enzyme, antibody is readily formed which recognizes the amino acid sequence different between mouse and human enzymes. Therefore, when mouse is immunized with human type II phospholipase A2, almost all mouse antibodies which bind to human type II phospholipase A2 are assumed theoretically not to bind to mouse type II phospholipase A2. Also, the enzyme active center and its vicinity of type II phospholipase A2 are generally preserved among various animal species, and the homology is fairly high between mouse and human. When mouse is immunized with human type II phospholipase A2, it is difficult to obtain, due to the immunological tolerance, antibodies which recognize the enzyme active center and its vicinity, resulting in the present situation where it is impossible to acquire antibodies with strong inhibitory activity to human type II phospholipase A2.

There has not been known any antibody which not only strongly inhibits human type II phospholipase A2, but also inhibits mouse type II phospholipase A2 and/or monkey type II phospholipase A2. Prior to the clinical test of new drugs on human subjects, it is a prerequisite to clarify their pharmaceutical effects in animal experiments. Therefore, it is necessary to have monoclonal antibody which shows pharmaceutical effectiveness in experimental animals, especially small ones such as mouse and monkey, that is, monoclonal antibody which inhibits not only human type II phospholipase A2, but also said enzyme from these other animals.

It is also known that phospholipase A2 must bind to cells when the enzyme exerts its hydrolytic action on membranous phospholipid (Suga H. et al., Eur. J. Biochem., 218: 807-813, 1993; Murakami M. et al., J. Biol. Chem., 268: 839-844, 1993). Accordingly, if antibodies are able to not only inhibit the free phospholipase A2 in blood stream or exudates, but also release said enzyme bound to cells, membranous phospholipids of which are to be hydrolyzed, these antibodies are expected to have potent inhibitory effects on phospholipase A2 based by a novel action mechanism. Furthermore, when antibody binds to phospholipase A2 associated with the cell surface, complements and/or effector cells may attack said cell, possibly exerting unfavorable side effects on it. However the antibodies which can release type II phospholipase A2 bound to the cell also have not been obtained yet.

Therefore, there has been a strong demand for antibodies against type II phospholipase A2 under consideration for pharmaceutics, wherein said antibodies strongly inhibit not only human type II phospholipase A2, but also said enzyme derived from other animal species, and also have properties to be capable of releasing type II phospholipase A2 bound to the cell.

Disclosure of the Invention

The present inventors made efforts to solve these problems described above, and obtained the following information to have accomplished the invention.

That is, we immunized mouse with a recombinant human type II phospholipase A2 mixed with Freund's complete adjuvant in a dose of 20 μg per mouse 8 times at the interval of 2 to 3 weeks. Thus, we differed from other researchers in that we intended to avoid the immunological tolerance by increasing the immunization frequency and extending the immunizing period of time. Furthermore, we tried to improve the system to assay the inhibition of phospholipase A2 for screening antibodies.

That is, we used phospholipid derived from *Escherichia coli* as a highly sensitive substrate for phospholipase A2 activity assay (Jacobson P. B. et al., Biochem. Pharm., 39: 1557, 1990), and also improved the method for preparing said *E. coli* phospholipids as the substrate. That is, we used *Escherichia coli* SN17 deficient in phospholipase A2 (pldA, pla-2, thr-1, leuB6, thi) (Doi 0. et al., J. Biochem., 80: 1247-1258, 1976), and incorporated tritium-labelled oleic acid into it to prepare its phospholipids. By using this microorganism, we were able to prepare the tritium-labelled *E. coli* phospholipid with a high specific radioactivity efficiently without exposing it to the degradation by *E. coli* phospholipase A2. Furthermore, by using an assay system with a longer enzyme reaction time and a higher sensitivity than the conventional method (J. Biol. Chem., 261: 4239-4246, 1986), we could efficiently and accurately screen the desired antibody-producing hybridoma. Thus, we examined their inhibitory effects on phospholipase A2 activity derived from various animals.

Also, in order to examine a novel function of antibodies thus obtained, we used an assay system for testing the capability of these antibodies to release phospholipase A2 bound to the cell.

As a result, we succeeded in obtaining a monoclonal antibody characteristic of having the following properties.

That is, the monoclonal antibody obtained by the present inventors is reactive with human type II phospholipase A2 and highly inhibitory against it. Also, the antibody is cross-reacting with type II phospholipase A2 derived from platelet of monkey and mouse. Therefore, by using the antibody of the present invention, animal experiments with monkey and mouse can be performed prior to the clinical test of new drugs in human. Furthermore, the monoclonal antibody of the present invention is able to not only inhibit the free enzyme in a blood stream or an exudate, but also release type II phospholipase A2 bound to the cell. Type II phospholipase A2 binds to the cell via heparin sulfate etc. on the cell surface, and is known to bind to hepatocyte, endothelial cell, etc. having heparin sulfate, etc. on the cell surface. Monoclonal antibody of the present invention is characteristic of not only inhibiting the free type II phospholipase A2 in a blood stream or an exudate, but also releasing the cell-bound enzyme from the cell membrane.

In addition, the present invention includes a protein consisting of a part of the monoclonal antibody with the properties described above and having the antigen-binding capability equivalent to that of said monoclonal antibody. Also, the present invention includes reduced alkylated derivatives of said protein, and variant proteins produced by the addition, deletion, substitution or mutation of one or more amino acid residues in the amino acid sequence of the monoclonal antibody obtained by immunizing animals, as long as they retain antigen-binding capability equivalent to that of said monoclonal antibody.

Furthermore, the present inventors isolated cDNAs encoding the variable regions of H and L chains of the antibody from "hybridoma 1.4" and "hybridoma 10.1" generating the antibody comprised in the present invention, and determined the entire base sequence. This enables not only the production of the antibody on a large scale using genetic engineering techniques, but also the ready modification of said antibody comprised in the present invention using antibody engineering techniques. [Base sequences determined with "hybridoma 1.4" are shown in SEQ ID NO: 1 (L chain) and SEQ ID NO: 3 (H chain). Also, putative amino acid sequences corresponding to said base sequences are shown in SEQ ID NO: 2 (L chain) and SEQ ID NO: 4 (H chain). Base sequences determined with "hybridoma 10.1" are shown in SEQ ID NO: 5 (L chain) and SEQ ID NO: 7 (H chain). Also, putative amino acid sequences corresponding to said base sequences are shown in SEQ ID NO: 6 (L chain) and SEQ ID NO: 8 (H chain).]

That is, the present invention comprises the followings;

1) A monoclonal antibody capable of inhibiting the activity of human type II phospholipase A2 as well as that of type II phospholipase A2 derived from monkey and/or mouse, or a protein comprising a part thereof having said inhibitory activity,
2) A monoclonal antibody capable of releasing type II phospholipase A2 bound to the cellular membrane, or a protein comprising a part thereof having said activity,
3) The monoclonal antibody or protein according to 2) wherein said phospholipase A2 is derived from human,
4) A monoclonal antibody capable of not only inhibiting the activity of human type II phospholipase A2 as well as that of type II phospholipase A2 derived from monkey and/or mouse, but also releasing type II phospholipase A2 bound to the cell membrane or a protein comprising a part thereof having said activity,
5) A monoclonal antibody produced by any one of hybridomas 12H5 (FERM BP-5300), 1.4 (FERM BP-5297) and 10.1 (FERM BP-5297) or a protein comprising a part thereof, or a monoclonal antibody or a protein comprising a part thereof having the activity against type II phospholipase A2 equivalent to that of said monoclonal antibody or protein,
6) The monoclonal antibody or protein comprising a part thereof according to 1) or 2), wherein said monoclonal antibody or protein comprises protein having the amino acid sequence represented by any one of SEQ ID NO:2, 4, 6 or 8 or those modified by substitution, deletion or insertion of one or more amino acid residues contained in said sequence,
7) The monoclonal antibody or protein comprising a part thereof according to 1) or 2), wherein said monoclonal antibody or protein comprises protein having the amino acid sequence represented by any one of SEQ ID NO:9 to 20 or those modified by substitution, deletion or insertion of one or more amino acid residues contained in said sequence,
8) A cell producing the monoclonal antibody or protein according to any one of 1) to 7),
9) The cell according to 8) wherein said cell is a hybridoma,
10) The cell according to 8) wherein said cell is a cell transformed with recombinant DNA,
11) A method for producing the monoclonal antibody or protein according to any one of 1) to 7), wherein said method comprises processes of culturing the cell according to 8) and recovering monoclonal antibodies or proteins from the supernatant of growth medium,
12) A DNA encoding the monoclonal antibody or protein according to any one of 1) to 7),
13) The DNA according to 12), wherein said DNA comprises the base sequence with any one of SEQ ID NO: 1, 3, 5 or 7, or that modified by substitution, deletion or insertion of one or more amino acid residues contained in said sequence,
14) A recombinant vector comprising the DNA according to 12) or 13),
15) A medical composition comprising the monoclonal antibody or protein according to any one of 1) to 7) and a pharmacologically acceptable carrier,
16) A type II phospholipase A2 inhibitor comprising the monoclonal antibody or protein according to any one of 1) to 7).

"Protein" of the present invention is not limited in number of amino acid residues, including so-called "peptide" with a fewer amino acid residues. Also, "cell producing a monoclonal antibody or protein" of the present invention includes every type of cells such as bacterium, yeast, mammalian cell, etc., so far as it is capable of producing the monoclonal antibody or protein of the present invention.

The monoclonal antibody of the present invention is produced by culturing mouse hybridoma in a growth medium or the mouse peritoneal fluid. Also, parts thereof such as F(ab')2, Fab, Fab', etc. may be obtained by digesting the produced antibody with a proteolytic enzyme selected from a group consisting of trypsin, papain and pepsin, followed by appropriate purification.

Hybridomas of the present invention, such as hybridomas 12H5, 1.4 and 10.1, may be obtained by fusing spleen cells of BALB/c strain mouse which has been sensitized with type II phospholipase A2 from normal humans in increased frequencies and for an elongated immunization period of time with mouse myeloma cells P3x63Ag8/U1(P3U1) by a standard method, for example, the cell fusion method described by Kohler and Milstein (see Example below).

Hybridomas obtained by the present inventors and included in the present invention are deposited as follows.

Deposition of hybridoma 12H5:

a) Name and address of depositary
Name: National Institute of Bioscience and Human-Technology, Agency of Industrial Science and Technology, Ministry of International Trade and Industry
Address: Higashi 1-1-3, Tsukuba-shi, Ibaragi-ken 305, Japan
b) Date of deposition (original date of deposition)
November 22, 1994
c) Accession No.
Seimeiken-Jo-Ki No. 5300 (FERM BP-5300)

Deposition of hybridoma 1.4:

a) Name and address of depositary
Name: National Institute of Bioscience and Human-Technology, Agency of Industrial Science and Technology, Ministry of International Trade and Industry
Address: Higashi 1-1-3, Tsukuba-shi, Ibaragi-ken 305, Japan
b) Date of deposition (original date of deposition)
November 22, 1994
c) Accession No.
Seimeiken-Jo-Ki No. 5297 (FERM BP-5297)

Deposition of hybridoma 10.1:

a) Name and address of depositary
Name: National Institute of Bioscience and Human-Technology, Agency of Industrial Science and Technology, Ministry of International Trade and Industry
Address: Higashi 1-1-3, Tsukuba-shi, Ibaragi-ken 305, Japan
b) Date of deposition (original date of deposition)
November 22, 1994
c) Accession No.
Seimeiken-Jo-Ki No. 5298 (FERM BP-5298)

As the culture medium of said hybridomas, Dalbecco's modified Eagle's minimum essential medium (abbreviated DMEM hereafter) supplemented with fetal calf serum, L-glutamine, glucose, sodium pyruvate, 2-mercaptoethanol and antibiotic (for example, penicillin G, streptomycin, gentamycin, etc.), etc. are used.

Hybridomas are usually cultured in the medium at 37°C in a 5% $CO_2$ - 95% air atmosphere for 2 to 4 days, or in the peritoneal cavity of BALB/c strain mouse pretreated with 2,6,10,14-tetramethylpentadecane (for example, pristan, Aldrich) for about 10 to 20 days to produce the antibody in an amount sufficient for purification.

In addition, the monoclonal antibody or protein comprising a part thereof of the present invention can be obtained by inserting all or part of gene encoding the antibody of the present invention or a part thereof into an expression vector, and transferring them into *E. coli*, yeast or mammalian cells, which will produce them.

The monoclonal antibody thus produced can be purified by fractionating the culture medium supernatant or peritoneal fluid by standard methods for isolating and purifying proteins. These methods include, for example, centrifugation, dialysis, ammonium sulfate precipitation, column chromatographies on DEAE-cellulose, hydroxylapatite, and protein-A agarose, etc.

From the antibody thus purified, its active fragments, for example, F(ab')2, Fab Fab' Fv can be obtained by digesting said antibody with proteolytic enzymes such as pepsin, papain, etc. followed by isolating and purifying the digest using standard methods. Furthermore, reduced alkylated derivatives of the antibody wherein H and L chains are linked only by the non-covalent bond can be obtained by reductive alkylation of disulfide bonds bridging H to H chain and/or H to L chain using dithiothreitol and iodoacetamide, etc. (Useful Immunological Experimental Method, p39, Kodansha Scientific Book).

In addition, in order to suppress the effector activity, it is possible to modify the amino acid sequence of Fc portion of the monoclonal antibody or a part thereof of the present invention (Duncan A. R. et al., Nature, 332: 738, 1988; Tao M. et. al., J. Exp. Med., 178: 661-667, 1993; Lund J., J. Immunology, 147: 2657, 1991). Also, it is possible to replace the Fc fragment with other protein. Further, it is possible to fuse Fc fragment with other protein to induce a novel function.

When the monoclonal antibody or protein comprising a part thereof of the present invention are used for human treatment, it is preferred to use the antibody or protein comprising a part thereof which contain human-derived portions in great proportions. Such monoclonal antibodies are exemplified by 1) so-called "chimeric antibody" consisting of the amino acid sequence derived from animal such as mouse, etc. only as the variable region and that derived from human as the constant region, and 2) so-called "humanized antibody" consisting of the amino acid sequence derived from animal such as mouse only in the complementary-determining region (abbreviated CDR hereafter, or hypervariable region) and that derived from human in the other regions. These antibodies of such types are also included in the present invention. These "chimeric antibody" or "humanized antibody" can be readily prepared by the ordinary skilled in the art by isolating the gene corresponding to the variable region or CDR from hybridoma of the present invention, re-combining it with the human antibody gene, and transducing the resulting gene into host cells to be expressed (see Int. J. Cancer, 44, 424-433 (1989); Proc. Natl. Acad. Sci. USA, 87, 8095-8099 (1990)). Also, once the base sequence of gene is determined, it is possible to use the gene comprising the corresponding sequence synthesized, or the gene obtained from hybridoma or human antibody-producing cells combined with said synthesized gene. Also, as the gene corresponding to the variable region or CDR, base sequences shown in any one of SEQ ID NO:1, 3, 5 or 7, or DNAs comprising the base sequence of their CDR regions are most preferable, or DNAs comprising base sequences encoding the amino acid sequence shown in any one of SEQ ID NO:2, 4, 6 or 8 or that of its CDR region, DNA genes comprising the base sequences prepared by modifying one or more bases of said DNA by substitution, deletion or insertion or parts thereof are preferably used. Mutagenesis of gene by substitution, deletion or insertion can be made by known methods (Gillamn et al., Gene, 8, 81-97 (1979); Roberts et al., Nature, 328, 731-734 (1987)). For screening variant base sequences thus obtained for the sequence encoding amino acid sequence having preferable properties, methods including phage display mothod (Ann. Rev. Immunol., 12, 433-455 (1994)) may be used. Especially, "humanized antibody" may be easily prepared by those skilled in the art using known method (Nature, 321, 522-525 (1986); Science, 239, 1534-1536 (1988); Proc. Natl. Acad. Sci. USA, 86, 10029-10033 (1989); Proc. Natl. Acad. Sci. USA, 88, 2869-2873 (1991)). Furthermore, according to these literatures, for the preparation of "humanized antibody", it is preferable to use human antibody which is highly homologous to that of animal other than human from which said CDR region to be transplanted is derived, as human antibody to be substituted in only CDR region with that derived from animal other than human (that is, antibody constituting the basic skeleton of "humanized antibody", called "human receptor antibody"). As shown in Example 7, human antibodies highly homologous to the mouse antibody obtained by the present inventors and included in the present invention were, for example, Pag-1 antibody (Hughes-Johns N. C. et al., Biochem. J., 268, 135-140 (1990)), WEA antibody (Goni F., et al., Proc. Natl. Acad. Sci. USA, 80, 4837-4841 (1983)); ITH5-2 antibody (Chin L. T., et al., Immunol. Letter, 44, 25-30 (1995)); ITC48 antibody (Ohlin M., et al., Mol. Immunol., 31, 983-991 (1994)), and these antibodies are conceivably preferable as "human receptor antibody" for preparing "humanized antibody".

"Chimeric antibody"may be easily prepared by those skilled in the art using known methods (Nature, 314, 268-270 (1985); Proc. Natl. Acad. Sci. USA, 84, 3439-3443 (1987); Proc. Natl. Acad. Sci. USA, 84, 214-218 (1987); Proc. Natl. Acad. Sci. USA, 81, 6851-6855 (1984)).

Preferred monoclonal antibody or proteins included in the present invention are exemplified by monoclonal antibodies 12H5 or 1.4 prepared by sensitizing animals with human type II phospholipase A2 as described above and their fragments such as F(ab')2, Fab, Fab', etc. Also, monoclonal antibodies comprising the variable or hypervariable regions of these monoclonal antibodies or their fragments and other regions derived from human are included.

Medical compositions of the present invention are effective especially by parenteral administration, that is, subcutaneous, intramuscular or intravenous administration. The composition for the parenteral administration is usually composed of a solution containing monoclonal antibody or protein comprising its fragment dissolved in an administrable carrier, preferably an aqueous carrier. Various aqueous carriers such as water, aqueous buffer, 0.4% saline, 0.3% glycinesolution, 5% glucose solution, human albumin solution, etc. can be used. These solutions should be sterilized, and generally contain no particle-forming materials. These compositions can be sterilized by conventional and well-known sterilization techniques. These compositions may contain pharmaceutically acceptable supplements to make them closer to the physiological conditions such as buffering and isotonic agents including, for example, sodium acetate, sodium chloride, potassium chloride, calcium chloride, sodium lactate, sodium citrate, etc. Actual preparation of parenterally administrable compositions can be performed by those skilled in the art using the established or well-known techniques, described, for example, in "Remington's Pharmaceutical Science, 15th edition, Mack Publishing Company, Easton, PA (1980)".

Monoclonal antibodies, proteins or medical compositions of the present invention can be stored frozen or lyophilized, and dissolved in appropriate solvent when used. Lyophilization and thawing and dissolution of the compositions can be performed by those skilled in the art using known methods.

<u>Brief Description of the Drawings</u>

Fig. 1 shows the result of ELISA for 12H5, 10.1 and 1.4 respectively with recombinant human type II phospholipase A2.

Fig. 2 shows the result of ELISA for 12H5, 10.1 and 1.4 respectively with phospholipase A2 derived from rat platelet.

Fig. 3 shows inhibition of 12H5, 10.1 and 1.4 respectively against recombinant human type II phospholipase A2 activity.

Fig. 4 shows inhibition of 12H5, 10.1 and 1.4 respectively against rat type II phospholipase A2 activity.

Fig. 5 shows inhibition of 12H5 against phospholipase A2 derived from platelets of various animals.

Fig. 6 shows inhibition of 10.1 against phospholipase A2 derived from platelets of various animals.

Fig. 7 shows inhibition of 1.4 against phospholipase A2 derived from platelets of various animals.

Fig. 8 shows the activity of 12H5, 10.1 and 1.4 respectively to release phospholipase A2 bound to BRL-3A cells.

Fig. 9 shows the anchored PCR method for cloning cDNA of variable regions of H and L chains of antibody 1.4.

Fig. 10 shows the cDNA sequence of L chain variable region of antibody 1.4 and the translated amino acid sequence. Putative sequences corresponding to CDR sequence are underlined.

Fig. 11 shows the cDNA sequence of H chain variable region of antibody 1.4 and the translated amino acid sequence. Putative sequences corresponding to CDR sequence are underlined.

Fig. 12 shows the method for cloning cDNA in the variable region of H and L chains of antibody by PCR.

Fig. 13 shows the cDNA sequence of L chain variable region of antibody 10.1 and the translated amino acid sequence. Putative sequences corresponding to CDR sequence are underlined.

Fig. 14 shows cDNA sequence of H chain variable region of antibody 10.1 and the translated amino acid sequence. Putative sequences corresponding to CDR sequence are underlined.

## Best mode for carrying out the Invention

In the following, Examples of the present invention are described without limiting the present invention by any means.

### Example 1 Preparation of hybridomas 12.5, 1.4 and 10.1

#### a) Preparation of recombinant human type II phospholipase A2

Monoclonal antibodies of the present invention can be obtained by immunizing animals with a recombinant human type II phospholipase A2, which was prepared entirely according to the method described in Tokkai H5-192167.

#### b) Preparation of sensitized spleen cells

Recombinant human type II phospholipase A2 purified in Example 1a) (20 μg per animal) was dissolved in the physiological saline (0.1 ml), mixed with an equal volume of Freund's complete adjuvant to emulsify, and peritoneally injected into BALB/c strain mouse (6-week-old at the initiation time of immunization) (first immunization). Then, an emulsion of the same amount of phospholipase A2 mixed with the equal volume of Freund's complete adjuvant was booster administered seven times at the interval of 2 to 3 weeks. Twenty four days after the 7th immunization, phospholipase A2 (20 μg per animal) dissolved in the physiological saline (0.2 ml) was peritoneally administered (final immunization). Three days after the final immunization, spleen cells were collected from one mouse, and suspended in DMEM medium.

#### c) Preparation of hybridomas 12H5, 1.4 and 10.1

Spleen cells prepared as above ($1 \times 10^8$ cells) were fused with mouse myeloma P3 x 63Ag8 • U1 (P3U1) ($2 \times 10^7$ cells) according to the method of Kohler and Milstein (see Nature, 256, 495 (1975)). That is, spleen cells and P3U1 cells were washed with DMEM several times, placed together in a 50-ml plastic centrifuge tube and thoroughly mixed. Then, after the medium was removed by centrifugation, DMEM (1 ml) which contained 50% (w/v) polyethylene glycol (Sigma, average molecular weight 3350) and pre-warmed to 37°C) was added slowly under stirring over 1 min.

Then, DMEM (10 ml) pre-warmed to 37°C was added dropwise to the above mixture to terminate fusing. The reaction mixture thus obtained was centrifuged to remove the supernatant, and to the residue was added a HAT medium [DMEM medium containing 10% fetal calf serum supplemented with hypoxanthine ($1 \times 10^{-4}$ M), aminopterin ($4 \times 10^{-7}$ M) and thymidine ($1.6 \times 10^{-5}$ M)] to adjust to concentration of $6 \times 10^5$ spleen cells/ml. This cell suspension was distributed in wells of a 96-well plastic plate at 200 μl per well ($1.2 \times 10^5$ spleen cells). Eleven days later, a half volume of the medium was removed by aspiration, and HAT medium described above was added to the residue. Seven to ten days after the cell fusion, the proliferation of hybridoma was observed in about half of wells. Activity of antibody in the supernatant was assayed by the method described in d) below. Positive clones were transfered to 48-well plastic plate, and thereafter to 24-well plate, where they were cultured in the same medium except for containing no aminopterin. Reproducibility of antibody activity was confirmed using the culture supernatant in a 24-well plastic plate by the method described in d), and at the same time the enzyme inhibitory activity of antibody was determined by the method described in e).

In order to ascertain that the three clones showing the enzyme inhibitory activity are monoclonal, successive cloning was performed by the limiting dilution method. Monoclonal antibodies generated by clones thus obtained were designated 12H5, 1.4 and 10.1, respectively.

d) Determination of antibody activity by ELISA

The purified recombinant human type II phospholipase A2 was dissolved in 20 mM Tris-buffered saline (TBS, pH 7.4) at 0.05 μg/ml. Aliquot (100 μl each) of the enzyme solution was placed in each well of a 96-well flat-bottomed microtiter plate and incubated in a humidified chamber at 4°C overnight. Then, after the solution was discarded, TBS containing 1% BSA (200 μl each) was added to each well, and left at 37°C for 1 h to block the unabsorbed fraction in each well. Then, each well was washed several times with TBS containing 0.05% Tween 20 (TBS-Tween). Supernatant of the culture medium for hybridoma culture or peritoneal cavity fluid, or a purified antibody preparation were diluted with TBS-Tween containing 0.2% BSA (BSA-TBS-Tween), and aliquot (100 μl each) of the solution was added to each well, and incubated at room temperature for 2 h.

After washing wells with TBS-Tween similarly as described above, horseradish peroxidase conjugated anti-mouse antibody (Zymed) diluted 2,000 times with BSA-TBS-Tween (100 μl each) was added to each well, and incubated at room temperature for 2 h. After the reaction, wells were washed with TBS-Tween similarly as above, and 0.1 M acetate-sodium citrate buffer (pH 5.8) (100 μl each) containing 0.006% hydrogen peroxide and 3,3',5,5'-tetramethylbenzidine (0.1 mg/ml) was added as the enzyme substrate to each well. After being left at room temperature for 30 min, 3 M sulfuric acid (25 μl each) was added to terminate the reaction, then the optical density of the reaction mixture was measured at 450 nm.

e) Assay of phospholipase A2 inhibiting activity

The inhibitory activity of antibody against phospholipase A2 was determined by a slight modification of the method described in Journal of Biological Chemistry (Pepinsky R. B. et al., J. Biol. Chem. 261(9), 4239-4246 (1986)) as follows.

A purified preparation of recombinant human type II phospholipase A2 (0.5 ng) and the supernatant of a hybridoma growth medium or a purified preparation of antibody was incubated in 125 mM Tris buffer (pH 8.0) (100 μl) containing 150 mM sodium chloride, 12.5 mM calcium chloride and bovine serum albumin (250 μg/ml) at room temperature for 2 h. Then, an autoclaved preparation of *Escherichia coli* SN17 into which tritium-labelled oleic acid had been incorporated was added to the reaction mixture (25 μl, 50,000 to 100,000 cpm), and the resulting mixture was incubated at 37°C for 30 min. The reaction mixture was transfered onto ice, and the reaction was terminated by adding 4 N hydrochloric acid (25 μl). After the reaction had been terminated, 25 μl of bovine serum albumin (40mg/ml) was added to the mixture and mixed ,and the resulting mixture was allowed to be on the ice for 30 minutes.

Then, after the centrifugation at 15,000 rpm for 2 min, radioactivity of the supernatant was measured. From the radioactivity thus obtained was subtracted that of the negative control (radioactivity of a sample containing neither phospholipase A2 nor antibody), the resulting value was used to express as the activity of phospholipase A2. Inhibitory ratio against antibody was expressed according to the following calculation based on the phospholipase A2 activity of the positive control (phospholipase A2 activity of a sample containing said enzyme but no antibody).

Inhibitory ratio against phospholipase A2 = {1 - (activity of phospholipase A2 incubated with the culture medium supernatant or purified antibody)/(activity of phospholipase A2 of positive control)} x 100

Autoclaved preparation of *E. coli* incorporated with tritium-labelled oleic acid as a substrate for the phospholipase A2 activity assay was prepared according to the method described in Tokkai H5-192167, p8.

Example 2 Generation and preparation of monoclonal antibody

a) Generation of monoclonal antibody

2,6,10,14-Tetramethylpentadecane (pristan, Sigma) (0.5 ml) was injected into the peritoneal cavity of BALB/c strain mouse (5 to 6 weeks after birth), and, 14 to 21 days later, hybridoma (5 x $10^6$) suspended in the physiological saline (0.5 ml) was peritoneally injected. Ten to twenty days later, peritoneal fluid produced was collected from the sacrificed and ventrotomized mouse. Peritoneal fluid containing monoclonal antibody was recovered amounting to 5 to 10 ml from one mouse.

b) Preparation of monoclonal antibody

After the peritoneal fluid was centrifuged to remove insoluble materials, equal volume of saturated ammonium sulfate solution was added, and the mixture was stirred for 1 h on ice. The precipitate formed was collected by centrifugation, dissolved in a small volume of 0.1 M phosphate buffer (p H 7.4) Containing 0.9% NaCl, and dialyzed against 100 volumes of the same buffer at 4°C overnight to obtain a crude gamma globulin fraction. From this fraction, IgG was purified using a MAPS-II mouse monoclonal antibody purification kit (BioRad Laboratories).

That is, to the gamma globulin fraction was added an equal volume of the binding buffer and mixed. The resulting mixture was loaded onto a column filled with "Protein A - Sepharose CL4B (Pharmacia)" which had been fully equilibrated with the same binding buffer (gel bed volume 20 ml), and the column was washed with 3 volumes of said binding buffer. IgG was eluted with about three column volumes of the elution buffer provided in the kit. IgG thus eluted was dialyzed against, for example, 20 mM Tris-buffered saline (pH 7.4) to obtain the antibody preparation. Usually, 5 to 10 mg of IgG per 1 ml of peritoneal fluid were obtained.

Example 3 Determination of subclass of antibodies

IgG subclass of antibodies in the supernatant of hybridoma culture medium was determined using a mouse monoclonal antibody typing kit (Amersham). By this method based on ELISA, subclasses of 12H5, 1.4 and 10.1 were determined to be IgG2a, IgG2a and IgG1, respectively.

Example 4 Cross reactivity of antibodies

a) Purification of rat type II phospholipase A2

To rat PRP (platelet-rich plasma) was added 1/5 volume of ACD solution (2% (w/v) glucose solution containing 65 mM citric acid and 85 mM sodium citrate), and the mixture was centrifuged at 2,500 x g for 10 min. To platelet thus precipitated was added ACD/F10 medium mixture (1:5) (F10, Gibco) to make a suspension containing 2 x $10^9$ platelets per ml, and the resulting suspension was centrifuged at 2500 x g for 10 min. Then, platelets thus precipitated were re-suspended in F10 medium to the density of 2 x $10^9$ cells per ml, and then to the resulting suspension were successively added 1 M calcium chloride and thrombin (2,500 unit/ml) to the final concentration of 2 mM and 2.5 unit, respectively.

The resulting mixture was incubated at 37°C for 5 min, centrifuged at 3,000 x g for 15 min to recover the supernatant (supernatant of rat platelet stimulated by thrombin), which was subjected to the later process. Phospholipase A2 was purified by a slight modification of the method described in Tokkai H5-192167, p12. That is, the supernatant of rat platelet stimulated by thrombin was passed through a column (Econocolumn, column size 20 ml; BioRad) of sulfated cellulofine (Seikagaku-Kogyo) which had been equilibrated with 0.1 M acetate buffer (pH 6.0). After the column was thoroughly washed with 200ml of washing buffer (0.1 M acetate buffer containing 0.5 M NaCl (pH 6.0)), it was eluted with 30ml of elution buffer (0.1 M acetate buffer containing 1.5 M NaCl (pH 6.0)) to obtain the sulfated cellulofine-binding fraction, which was further fractionated by HPLC.

HPLC was performed in a LC-4A HPLC system (Shimazu Seisaku-sho) using a CAPCELL PAK C18 column (4.6 mm ID x 25 cm, Shiseido) all at the flow rate of 1 ml/min. The sulfated cellulofine-binding fraction was passed through the column, which was then washed by passing 0.1% trifluoroacetic acid for 60 min. Then, phospholipase A2 was eluted with a linear gradient of acetonitrile from 0 to 50% in presence of 0.1% trifluoroacetic acid. Phospholipase A2 eluted with a peak around 30% acetonitrile was recovered.

On SDS-polyacrylamide gel electrophoresis, the fraction thus recovered showed a single protein band by silver stain, confirming this fraction as rat type II phospholipase A2.

b) Preparation of type II phospholipase A2 derived from platelet of human, monkey, dog, rabbit, cat and mouse

Type II phospholipase A2 derived from platelet of various animal species was purified according to the method for preparing sonicate of human platelet described in Journal Biological Chemistry (J. Biol. Chem., 264 (10), 5768-5775 (1989)).

To PRP (platelet-rich plasma) prepared from human, rhesus monkey, dog, rabbit, mouse and cat was added EDTA to a final concentration of 2 mM, and the mixture was centrifuged at 2500 x g for 10 min to recover cells. Cells were suspended in 30 mM Tris-HCl buffer containing 120 mM NaCl and 2 mM EDTA (pH 7.4), and centrifuged again at 2,500 x g for 10 min. After repeating this procedure twice, cells thus obtained were re-suspended in 30 mM Tris-HCl buffer containing 120 mM NaCl and 2 mM EDTA (pH 7.4) to a density of 2 x $10^9$ cells/ml, and frozen in liquid nitrogen.

Frozen cell suspension was thawed, mixed with an equal volume of 0.36 N sulfuric acid, and the mixture was sonicated using a BIOMC 7040 ULTRA SONIC PROCESSOR (Seiko; output 80, 15 s, 6 times). Cell sonicate thus obtained was left on ice for 60 min, and then centrifuged at 4°C and 10,000 x g for 30 min to recover the supernatant. Precipitate was re-suspended in 0.18 N sulfuric acid the volume of which was 1/3 volume of the supernatant, left on ice for 60 min, and then centrifuged at 4°C and 10,000 x g for 30 min to recover the supernatant. Both supernatants were combined, and dialyzed against 50 mM acetate buffer containing 200 mM NaCl (pH 4.5) overnight. Dialyzed sonicate was centrifuged at 4°C and 15,000 x g for 40 min to recover the supernatant, which was used as phospholipase A2 derived from platelet.

c) ELISA reactivity

Purified preparations of various monoclonal antibodies obtained in Example 2b) were subjected to the following experiments.

IgG preparation was adjusted to a concentration of 10 μg/ml with BSA-TBS-Tween, and serially diluted 3 times. ELISA was performed with these serially diluted preparations according to Example 1d) to examine the cross-reactivity toward recombinant human type II phospholipase A2 and rat type II phospholipase A2. ELISA for rat type II phospholipase A2 was performed by replacing recombinant human type II phospholipase A2 in Example 1d) with rat type II phospholipase A2.

Results are shown in Fig. 1 and 2 with the concentration of antibody shown on abscissa and the absorption at 450 nm on ordinate. Cross reactivity toward rat phospholipase A2 was observed with 12H5 and 1.4, but not with 10.1.

d) Inhibitory activity against phospholipase A2 derived from various animals

Purified preparations of various monoclonal antibodies obtained in Example 2b) were subjected to the following experiments.

1) Inhibitory activity against recombinant human type II phospholipase A2

Using recombinant human type II phospholipase A2 described in Actual Example 1a), and according to Example 1e), the inhibitory activity of antibodies against phospholipase A2 was determined. Inhibitory ratios of each antibody toward recombinant human type II phospholipase A2 are shown in Fig. 3 with concentrations of antibody samples pre-incubated with phospholipase A2 shown on abscissa. 12H5 and 1.4 almost completely inhibited phospholipase A2 activity at a concentration of 10 μg/ml. On the other hand, the inhibitory ratio of 10.1 reached 80% at a concentration of 3 μg/ml.

2) Inhibitory activity against rat type II phospholipase A2

Inhibitory activity to rat type II phospholipase A2 was assayed by replacing recombinant human type II phospholipase A2 in Example 1e) with the purified preparation of rat type II phospholipase A2 (0.5 ng) in Actual Example 4a). Inhibitory ratios of each antibody to rat type II phospholipase A2 are shown in Fig. 4 with concentrations of antibodies pre-incubated with phospholipase A2 sample shown on abscissa. 12H5 and 1.4 also inhibited rat type II phospholipase A2, and the concentration of antibody required for a 50% inhibition of phospholipase A2 activity was 5 μg/ml with 12H5.

3) Inhibitory activity against type II phospholipase A2 derived from platelet of human, monkey, dog, rabbit, cat and mouse

Inhibitory activity against phospholipase A2 was assayed by replacing the recombinant human type II phospholipase A2 in Actual Example 1e) with a predetermined amount (20 to 40 μl) of each platelet sonicate. Inhibitory ratios of each antibody against phospholipase A2 are shown in Figs. 5 to 7 with concentrations of antibodies pre-incubated with platelet sonicate shown on abscissa. 12H5, 1.4 and 10.1 inhibited phospholipase A2 derived from human platelet similarly as the recombinant human type II phospholipase A2, indicating that antibody against the recombinant human type II phospholipase A2 of the present invention had the activity to inhibit the natural human type II phospholipase A2. On the other hand, antibodies of the present invention inhibited phospholipase A2 derived from platelet of rhesus monkey and mouse, partially inhibited that derived from platelet of dog, but not that derived from platelet of rabbit and cat.

Example 5 Activity of antibodies to release type II phospholipase A2 bound to cell

Hepatocyte (BRL-3A, purchased from Dainippon Pharmaceutical) was cultured on a 96-well flat-bottomed plate (Falcon Co.) using F12K/10% FCS (F12K, Dainihon Pharmaceutical Co.). After the culture medium of cells grown to confluence was removed, F12k/10% FCS (50 μl) containing $^{125}$I-labelled recombinant human type II phospholipase A2 (4 ng) was added to the cells in wells, and incubated at 37°C to bind phospholipase A2 to the cells. After 1 h, F12K/10% FCS containing a purified preparation of antibody or F12K/10% FCS (50 μl each) alone was added to wells and incubated for another 2 h. After the incubation, the culture medium was recovered to measure its radioactivity. At the same time, radioactivity of $^{125}$I-labelled phospholipase A2 (4 ng) which was added to the reaction system was separately measured.

Activity of antibody to release phospholipase A2 from the cell was expressed as percentage of the radioactivity of the medium incubated with various concentrations of antibody, per radioactivity of phospholipase A2 added minus radioactivity of the medium which F12K/10% FCS was added to only after cell binding to phospholipase A2 and were incubated.

Recombinant human type II phospholipase A2 was labelled with $^{125}$I by the following method. To 100 mM Tris-HCl buffer (pH 7.4) (100 μl) containing recombinant human type II phospholipase A2 (30 μg) placed in a glass test tube coated with IODOGEN (10 μg, Pierce) was added 6 μl of Na$^{125}$I (100 mCi/ml, ICN), and the mixture was left at room temperature for 20 min. The reaction was terminated by taking the reaction mixture out of the tube, and unreacted Na$^{125}$I was removed by gel filtration.

Phospholipase A2-bound cells were incubated with varied concentrations of purified antibody preparation, and radioactivity of the incubation medium was measured. Activity of each antibody to release cell-bound phospholipase A2 is shown in Fig. 8 with concentration of antibody added in the medium shown on abscissa. Mouse antibody (control) did not release phospholipase A2 from the cell at all, while 12H5, 10.1 and 1.4 concentration-dependently released said enzyme from the cell.

Example 6 Cloning of cDNA in the variable region of H and L chains of antibody

Messenger RNA for cDNA cloning was prepared from about $10^7$ hybridoma cells using a QuickPrep Micro mRNA Purification Kit (Pharmacia). That is, cells were suspended in Extraction buffer, from which mRNA was isolated using Oligo(dT)-Cellulose.

cDNA corresponding to the variable region of H and L chains of antibody 1.4 was prepared using 5' RACE System Kit (Gibco) according to the procedure shown in Fig. 9. That is, using primers which hybridize with constant region gene (C) and reverse transcriptase (SUPER SCRIPT II Reverse Transcriptase) with messenger RNA (1 μg) as template, single stranded cDNA was synthesized. A set of primers used were [GSP1L (5'-GGCACCTCCAGATGTTAACTGC-3')/SEQ ID NO: 21] for L chain, and [GSP1H (5'-GGAA(AG)TA(AGC)CCCTTGACCAGGC-3')/SEQ ID NO: 22] for H chain. Sequences in parentheses in that of GSP1H represent bases corresponding to degeneracy. Then, after mRNA which was a template was digested with RNase H, single-stranded cDNA was purified using "GLASS MAX Spin Cartridge". Poly(dC) tail was linked to 3'-terminus of the single-stranded cDNA using dCTP and terminal deoxynucleotide transferase. Variable region genes (V) of H and L chains were amplified using anchor primer which hybridizes with poly(dC) tail, (5'-CUACUACUACUAGGCCACGCGTCGACTAGTACGGGIIGGGIIGGGIIG-3'/SEQ ID NO: 23) and primer which hybridizes with constant region gene (C), [GSP2L (5'-TATAGAGCTCAAGCTTGGATGGTGGGAAGATGGATACAGTTGGTGC-3')/SEQ ID NO: 24] in the case of L chain, or [GSP2H (5'-TATAGAGCTCAAGCTTCCAGTGGATAGAC(CAT)GATGGGG(GC)TGT(TC)GTTTTGGC-3')/SEQ ID NO: 25] in the case of H chain with LA Taq (Takara Shuzo). Sequences in parentheses in that of GSP2H represent bases corresponding to degeneracy. On 1.2% agarose gel electrophoresis, these PCR products showed single bands of both H and L chains corresponding to around 550 to 570 base pairs.

Also, cDNAs corresponding to variable regions of H and L chains of antibody 10.1 were prepared according to the procedure shown in Fig. 12 using a Marathon cDNA Amplification Kit (Clontec). That is, single-stranded cDNA was synthesized using a Marathon cDNA synthesis primer and a reverse transcriptase with messenger RNA (1 μg) as template. This reaction solution was further reacted with RNase H, DNA polymerase I and DNA ligase to synthesize double stranded cDNA. Then, this reaction solution was reacted with T4 DNA polymerase to blunt the terminus of double-stranded cDNA. Marathon cDNA adapter was linked to double stranded cDNA using T4 DNA ligase. Variable region genes (V) of H and L chains were amplified using adapter primer 1 which hybridizes with adapter and primer GSP2L (in the case of L chain) or GSP2H (in the case of H chain) with LATaq (Takara Shuzo). On 1.2% agarose gel electrophoresis, these PCR products showed single bands corresponding to both H and L chains around 550 to 570 base pairs.

For sequencing, fragments amplified by PCR were integrated into pCR™II vector using TA Cloning Kit (Invitrogen). Competent cells of *Escherichia coli* JM109 (Takara-shuzo) were transformed with the fragment-inserted pCR™II plasmid. Using colonies formed on the plate and [5' primer UD (5'-ACCGAGCTCGGATCCACTAG-3')/SEQ ID NO: 26] which hybridizes with pCR™II, and [3' primer DU (5'-ATGCATGCTCGAGCGGCCGCC-3')/SEQ ID NO: 27], colony PCR was performed with Taq polymerase (Takara-shuzo). By analyzing amplified DNA fragments on 1.2% agarose gel electrophoresis, out of colonies comprising amplified fragments of about 600 to 620 base pairs were selected three clones for L chain and four clones for H chain of antibody 1.4, and two clones for L chain and four clones for H chain of antibody 10.1. *E. coli* of each clone was cultured, and plasmid DNA was prepared using QIAwell-8 Plasmid Purification Kit (Qiagen).

Using plasmid DNA prepared from each clone, and primer UD, primer DU, GSP2L for L-chain, or GSP2H for H chain with a DNA Sequencing Kit (Parkin-Elmer), sequencing reaction was performed, and analyzed by a 373DNA Sequencer (ABI). Three L chain specific and four H chain specific clones of antibody 1.4, and two L chain specific and four H chain specific clones comprised the same sequence, respectively. Fig. 10 (SEQ ID NO:1 and 2) concerning L chain of antibody 1.4 and Fig. 11 (SEQ ID NO:3 and 4) concerning H chain of the same antibody show cDNA sequence in the variable region and its putative amino acid sequence, respectively. Fig. 13 (SEQ ID NO:5 and 6) concerning L chain of antibody 10.1 and Fig. 14 (SEQ ID NO:7 and 8) concerning H chain of the same antibody show cDNA sequence in variable region and its putative amino acid sequence. In each figure, putative sequence corresponding to CDR sequence is underlined. (Putative CDR sequences of L chain of antibody 1.4, H chain of antibody 1.4, L chain of antibody 10.1 and H chain of antibody 10.1 are shown under SEQ ID NO:9 to 11, SEQ ID NO:12 to 14, SEQ ID NO:15 to 17, and SEQ ID NO:18 to 20, respectively.)

Example 7 Search for Homology of Amino acid Sequence in Variable Region of H and L Chains between Antibodies 1.4 and 10.1 and Human Antibody

Human antibody having a high homology to the amino acid sequences of variable regions of H and L chains of antibodies 1.4 and 10.1 obtained in Example 6 was searched in GenBank, EMBL, SWISS-PROT, PIR and PRF data bases. Homology search was performed using a BLASTP 1.4.8MP (Altschul S. F. et al., J. Mol. Biol., 215, 403-410 (1990)) program. As a result, antibody 1.4 was found to be highly homologous to, for example, Pag-1 antibody (Hughes-Jones N. C. et al., Biochem. J., 268, 135-140 (1990)) and WEA antibody (Goni F. et al., Proc. Natl. Acad. Sci. USA, 80, 4837-4841 (1983)). Also, it was found that antibody 10.1 had a high homology to, for example, ITH5-2 antibody (Chin L. T. et al., Immunol. Letter, 44, 25-30 (1995)) and ITC48 antibody (Ohlin M., et al., Mol. Immunol., 31, 983-991 (1994)).

Industrial Applicability

Monoclonal antibody and protein comprising a part thereof of the present invention are reactive with human type II phospholipase A2, and highly inhibitory against said enzyme. Since said antibody and protein are cross-reactive with type II phospholipase A2 derived from platelet of monkey and/or mouse, and can be applied to these experimental animals, they may be advantageously used in the animal experiment with monkey and/or mouse prior to pharmacological test in humans. In addition, since monoclonal antibody and protein comprising a part thereof of the present invention are capable of releasing type II phospholipase A2 bound to the cell, they are especially inhibitory to type II phospholipase A2, and supposedly not inducing side effects caused by attack of complement and/or effector cell.

Monoclonal antibody and protein comprising a part thereof of the invention can be applied as therapeutic to various diseases which are conceived to be caused or exacerbated by human type II phospholipase A2 (including cardiac infarction, cerebral infarction, acute renal insufficiency, allergic disease such asthma, chronic rheumatism, osteoarthritis, septic shock, pancreatitis, psoriasis, multiple organ failure (MOF), acute respiratory distress syndrome (ARDS), Crohn's disease and ulcerative colitis, uveitis, respiratory distress syndrome of the newborn (RDS), bronchopulmonary dysplasia (BPD), etc.).,

SEQUENCE LISTING

INFORMATION FOR SEQ ID NO:1

SEQUENCE CHARACTERISTICS:

LENGTH: 393

TYPE: nucleic acid

STRANDEDNESS: double

TOPOLOGY: linear

MOLECULE TYPE: cDNA to mRNA

ORIGINAL SOURCE:

CELL LINE: 1.4

FEATURE:

NAME/KEY: CDS

LOCATION: 1 .. 393

IDENTIFICATION METHOD: E

SEQUENCE DESCRIPTION: SEQ ID NO:1:

```
ATG GAG ACA GAC ACA CTC CTG CTA TGG GTG CTG CTG CTC TGG GTT CCA     48
Met Glu Thr Asp Thr Leu Leu Leu Trp Val Leu Leu Leu Trp Val Pro
  1               5                  10                  15
GGT TCC ACA GGT GAC ATT GTG CTG ACC CAA TCT CCA GCT TCC TTG GCT     96
Gly Ser Thr Gly Asp Ile Val Leu Thr Gln Ser Pro Ala Ser Leu Ala
```

20 25 30

GTG TCT TTA GGG CAG AGG GCC ACC ATA TCC TGC AGA GCC AGT GAA AGT 144

Val Ser Leu Gly Gln Arg Ala Thr Ile Ser Cys Arg Ala Ser Glu Ser

35 40 45

GTT GAT AGT TAT GGC ATT AGT TTT ATG CAC TGG TAT CAG CAG AAA CCA 192

Val Asp Ser Tyr Gly Ile Ser Phe Met His Trp Tyr Gln Gln Lys Pro

50 55 60

GGA CAG CCC CCC AAA CTC CTC ATT TAT CGT GCA TCC AAC CTA GAA TCT 240

Gly Gln Pro Pro Lys Leu Leu Ile Tyr Arg Ala Ser Asn Leu Glu Ser

65 70 75 80

GGG ATC CCT GCC AGG TTT AGT GGC AGT GGG TCT AGG ACA GAA TTC ACC 288

Gly Ile Pro Ala Arg Phe Ser Gly Ser Gly Ser Arg Thr Glu Phe Thr

85 90 95

CTC ACC ATT AAT CCT GTG GAG GCT GAT GAT GTT GCA ACC TAT CAC TGT 336

Leu Thr Ile Asn Pro Val Glu Ala Asp Asp Val Ala Thr Tyr His Cys

100 105 110

CAG CAA AGT AAT GAG GAT CCA TTC ACG TTC GGC TCG GGG ACA AAG TTG 384

Gln Gln Ser Asn Glu Asp Pro Phe Thr Phe Gly Ser Gly Thr Lys Leu

115 120 125

GAA ATA AAA 393

Glu Ile Lys

130

INFORMATION FOR SEQ ID NO:2

SEQUENCE CHARACTERISTICS:

LENGTH: 131

TYPE: amino acid

TOPOLOGY: linear

MOLECULE TYPE: protein

ORIGINAL SOURCE:

CELL LINE: 1.4

SEQUENCE DESCRIPTION: SEQ ID NO:2:

```
Met Glu Thr Asp Thr Leu Leu Leu Trp Val Leu Leu Leu Trp Val Pro
 1               5                  10                  15
Gly Ser Thr Gly Asp Ile Val Leu Thr Gln Ser Pro Ala Ser Leu Ala
            20                  25                  30
Val Ser Leu Gly Gln Arg Ala Thr Ile Ser Cys Arg Ala Ser Glu Ser
        35                  40                  45
Val Asp Ser Tyr Gly Ile Ser Phe Met His Trp Tyr Gln Gln Lys Pro
    50                  55                  60
Gly Gln Pro Pro Lys Leu Leu Ile Tyr Arg Ala Ser Asn Leu Glu Ser
65                  70                  75                  80
Gly Ile Pro Ala Arg Phe Ser Gly Ser Gly Ser Arg Thr Glu Phe Thr
                85                  90                  95
Leu Thr Ile Asn Pro Val Glu Ala Asp Asp Val Ala Thr Tyr His Cys
            100                 105                 110
Gln Gln Ser Asn Glu Asp Pro Phe Thr Phe Gly Ser Gly Thr Lys Leu
        115                 120                 125
```

Glu Ile Lys

130

INFORMATION FOR SEQ ID NO:3

SEQUENCE CHARACTERISTICS:

LENGTH: 408

TYPE: nucleic acid

STRANDEDNESS: double

TOPOLOGY: linear

MOLECULE TYPE: cDNA to mRNA

ORIGINAL SOURCE:

CELL LINE: 1.4

FEATURE:

NAME/KEY: CDS

LOCATION: 1 .. 408

IDENTIFICATION METHOD: E

SEQUENCE DESCRIPTION: SEQ ID NO:3:

```
ATG AGA GTG CTG ATT CTT TTG TGG CTG TTC ACA GCC TTT CCT GGT TTC      48
Met Arg Val Leu Ile Leu Leu Trp Leu Phe Thr Ala Phe Pro Gly Phe
  1               5                  10                  15
```

CTG TCT GAT GTG CAG CTT CAG GAA TCG GGA CCT GGC CTG GTG AAA CCT 96
Leu Ser Asp Val Gln Leu Gln Glu Ser Gly Pro Gly Leu Val Lys Pro
20 25 30

TCT CAA TCT CTG TCC CTC ACC TGC ATG GTC ACT GGC TAC TCA ATC ACC 144
Ser Gln Ser Leu Ser Leu Thr Cys Met Val Thr Gly Tyr Ser Ile Thr
35 40 45

AGT GAT TAT GCC TGG AAC TGG ATC CGG CAG TTT CCG GGA AAC AAA CTG 192
Ser Asp Tyr Ala Trp Asn Trp Ile Arg Gln Phe Pro Gly Asn Lys Leu
50 55 60

GAG CGG ATG GGA TAC ATA AGG TAC AGT GGT TAC ACT AGC TAC AAC CCA 240
Glu Arg Met Gly Tyr Ile Arg Tyr Ser Gly Tyr Thr Ser Tyr Asn Pro
65 70 75 80

TCT CTC AAA AGT CGA ATC TTT ATC ACG CGA GAC ACA TCC CAG AAC CAG 288
Ser Leu Lys Ser Arg Ile Phe Ile Thr Arg Asp Thr Ser Gln Asn Gln
85 90 95

TTC TTC CTA CAT TTG ACT TCT GTG ACT ACT GAG GAC ACA GCC ACA TAT 336
Phe Phe Leu His Leu Thr Ser Val Thr Thr Glu Asp Thr Ala Thr Tyr
100 105 110

TAC TGT ACA AGA GAC TTG GAC GCC TGG TAC TTC GAT GTT TGG GGC GCA 384
Tyr Cys Thr Arg Asp Leu Asp Ala Trp Tyr Phe Asp Val Trp Gly Ala
115 120 125

GGG ACA ACG GTC ACC GTC TCC TCA 408
Gly Thr Thr Val Thr Val Ser Ser
130 135

INFORMATION FOR SEQ ID NO:4

SEQUENCE CHARACTERISTICS:

LENGTH: 136

TYPE: amino acid

TOPOLOGY: linear

MOLECULE TYPE: protein

ORIGINAL SOURCE:

CELL LINE: 1.4

SEQUENCE DESCRIPTION: SEQ ID NO:4:

```
Met Arg Val Leu Ile Leu Leu Trp Leu Phe Thr Ala Phe Pro Gly Phe
 1               5                  10                  15
Leu Ser Asp Val Gln Leu Gln Glu Ser Gly Pro Gly Leu Val Lys Pro
            20                  25                  30
Ser Gln Ser Leu Ser Leu Thr Cys Met Val Thr Gly Tyr Ser Ile Thr
        35                  40                  45
Ser Asp Tyr Ala Trp Asn Trp Ile Arg Gln Phe Pro Gly Asn Lys Leu
    50                  55                  60
Glu Arg Met Gly Tyr Ile Arg Tyr Ser Gly Tyr Thr Ser Tyr Asn Pro
65                  70                  75                  80
Ser Leu Lys Ser Arg Ile Phe Ile Thr Arg Asp Thr Ser Gln Asn Gln
                85                  90                  95
Phe Phe Leu His Leu Thr Ser Val Thr Thr Glu Asp Thr Ala Thr Tyr
            100                 105                 110
```

Tyr Cys Thr Arg Asp Leu Asp Ala Trp Tyr Phe Asp Val Trp Gly Ala

115 120 125

Gly Thr Thr Val Thr Val Ser Ser

130 135

INFORMATION FOR SEQ ID NO:5

SEQUENCE CHARACTERISTICS:

LENGTH: 381

TYPE: nucleic acid

STRANDEDNESS: double

TOPOLOGY: linear

MOLECULE TYPE: cDNA to mRNA

ORIGINAL SOURCE:

CELL LINE: 10.1

FEATURE:

NAME/KEY: CDS

LOCATION: 1 .. 381

IDENTIFICATION METHOD: E

SEQUENCE DESCRIPTION: SEQ ID NO:5:

ATG GAA TCA CAG ACT CTG GTC TTC ATA TCC ATA CTG CTC TGG TTA TAT 48

Met Glu Ser Gln Thr Leu Val Phe Ile Ser Ile Leu Leu Trp Leu Tyr
1 5 10 15

GGA GCT GAT GGG AAC ATT GTA ATG ACC CAA TCT CCC AAA TCC ATG TCC 96
Gly Ala Asp Gly Asn Ile Val Met Thr Gln Ser Pro Lys Ser Met Ser
20 25 30

ATG TCA GTA GGA GAG AGG GTC ACC TTG ACC TGC AAG GCC AGT GAG AAT 144
Met Ser Val Gly Glu Arg Val Thr Leu Thr Cys Lys Ala Ser Glu Asn
35 40 45

GTG GTT ACT TAT GTT TCC TGG TAT CAA CAG AAA CCA GAG CAG TCT CCT 192
Val Val Thr Tyr Val Ser Trp Tyr Gln Gln Lys Pro Glu Gln Ser Pro
50 55 60

AAA CTG CTG ATA TAC GGG GCA TCC AAC CGG TAC ACT GGG GTC CCC GAT 240
Lys Leu Leu Ile Tyr Gly Ala Ser Asn Arg Tyr Thr Gly Val Pro Asp
65 70 75 80

CGC TTC ACA GGC AGT GGA TCT GCA ACA GAT TTC ACT CTG ACC ATC AGC 288
Arg Phe Thr Gly Ser Gly Ser Ala Thr Asp Phe Thr Leu Thr Ile Ser
85 90 95

AGT GTG CAG GCT GAA GAC CTT GCA GAT TAT CAC TGT GGA CAG GGT TAC 336
Ser Val Gln Ala Glu Asp Leu Ala Asp Tyr His Cys Gly Gln Gly Tyr
100 105 110

AGC TAT CCG TAC ACG TTC GGA GGG GGG ACC AAG CTG GAA ATA AAA 381
Ser Tyr Pro Tyr Thr Phe Gly Gly Gly Thr Lys Leu Glu Ile Lys
115 120 125

INFORMATION FOR SEQ ID NO:6

SEQUENCE CHARACTERISTICS:

LENGTH: 127

TYPE: amino acid

TOPOLOGY: linear

MOLECULE TYPE: protein

ORIGINAL SOURCE:

CELL LINE: 10.1

SEQUENCE DESCRIPTION: SEQ ID NO:6:

```
Met Glu Ser Gln Thr Leu Val Phe Ile Ser Ile Leu Leu Trp Leu Tyr
  1               5                  10                  15
Gly Ala Asp Gly Asn Ile Val Met Thr Gln Ser Pro Lys Ser Met Ser
             20                  25                  30
Met Ser Val Gly Glu Arg Val Thr Leu Thr Cys Lys Ala Ser Glu Asn
         35                  40                  45
Val Val Thr Tyr Val Ser Trp Tyr Gln Gln Lys Pro Glu Gln Ser Pro
     50                  55                  60
Lys Leu Leu Ile Tyr Gly Ala Ser Asn Arg Tyr Thr Gly Val Pro Asp
 65                  70                  75                  80
Arg Phe Thr Gly Ser Gly Ser Ala Thr Asp Phe Thr Leu Thr Ile Ser
                 85                  90                  95
Ser Val Gln Ala Glu Asp Leu Ala Asp Tyr His Cys Gly Gln Gly Tyr
            100                 105                 110
Ser Tyr Pro Tyr Thr Phe Gly Gly Gly Thr Lys Leu Glu Ile Lys
```

115 120 125

INFORMATION FOR SEQ ID NO:7

SEQUENCE CHARACTERISTICS:

LENGTH: 414

TYPE: nucleic acid

STRANDEDNESS: double

TOPOLOGY: linear

MOLECULE TYPE: cDNA to mRNA

ORIGINAL SOURCE:

CELL LINE: 10.1

FEATURE:

NAME/KEY: CDS

LOCATION: 1 .. 414

IDENTIFICATION METHOD: E

SEQUENCE DESCRIPTION: SEQ ID NO:7:

```
ATG GCT GTC CTG GCA TTA CTT TTT TGC CTG GTA ACA TTC CCA AGC TGT      48
Met Ala Val Leu Ala Leu Leu Phe Cys Leu Val Thr Phe Pro Ser Cys
  1               5                  10                  15
ATC CTT TCC CAG GTG CAG CTG AAG GAG TCA GGA CCT GGC CTG GTG GCG      96
```

Ile Leu Ser Gln Val Gln Leu Lys Glu Ser Gly Pro Gly Leu Val Ala

20 25 30

CCC TCA CAG AGC CTG TCC ATC ACA TGT ACC GTC TCA GGG TTC TCA TTA 144

Pro Ser Gln Ser Leu Ser Ile Thr Cys Thr Val Ser Gly Phe Ser Leu

35 40 45

ACC GAC TTT GGT GTA AAC TGG GTT CGC CAG CCT CCA GGA AAG GGT CTG 192

Thr Asp Phe Gly Val Asn Trp Val Arg Gln Pro Pro Gly Lys Gly Leu

50 55 60

GAG TGG CTG GGA ATG ATA TGG ACT GAT GGA ATC ACA GAC TAT AAT TCA 240

Glu Trp Leu Gly Met Ile Trp Thr Asp Gly Ile Thr Asp Tyr Asn Ser

65 70 75 80

GTT CTC AAA TCC AGA CTG AGC ATC AGC AAG GAC ACC TCC AAG AGC CAA 288

Val Leu Lys Ser Arg Leu Ser Ile Ser Lys Asp Thr Ser Lys Ser Gln

85 90 95

GTT TTC TTG AAA ATG AAC AAT CTG CAA ACT GAT GAC ACA GCC AGG TAC 336

Val Phe Leu Lys Met Asn Asn Leu Gln Thr Asp Asp Thr Ala Arg Tyr

100 105 110

TAC TGT GCC AGA GAT GCA TAC TAC GGC TTC TAT GCT ATG GAC TAC TGG 384

Tyr Cys Ala Arg Asp Ala Tyr Tyr Gly Phe Tyr Ala Met Asp Tyr Trp

115 120 125

GGT CAA GGA ACC TCA GTC ACC GTC TCC TCA 414

Gly Gln Gly Thr Ser Val Thr Val Ser Ser

130 135

INFORMATION FOR SEQ ID NO:8

SEQUENCE CHARACTERISTICS:

LENGTH: 138

TYPE: amino acid

TOPOLOGY: linear

MOLECULE TYPE: protein

ORIGINAL SOURCE:

CELL LINE: 10.1

SEQUENCE DESCRIPTION: SEQ ID NO:8:

```
Met Ala Val Leu Ala Leu Leu Phe Cys Leu Val Thr Phe Pro Ser Cys
 1               5                  10                  15
Ile Leu Ser Gln Val Gln Leu Lys Glu Ser Gly Pro Gly Leu Val Ala
            20                  25                  30
Pro Ser Gln Ser Leu Ser Ile Thr Cys Thr Val Ser Gly Phe Ser Leu
        35                  40                  45
Thr Asp Phe Gly Val Asn Trp Val Arg Gln Pro Pro Gly Lys Gly Leu
    50                  55                  60
Glu Trp Leu Gly Met Ile Trp Thr Asp Gly Ile Thr Asp Tyr Asn Ser
65                  70                  75                  80
Val Leu Lys Ser Arg Leu Ser Ile Ser Lys Asp Thr Ser Lys Ser Gln
                85                  90                  95
Val Phe Leu Lys Met Asn Asn Leu Gln Thr Asp Asp Thr Ala Arg Tyr
            100                 105                 110
Tyr Cys Ala Arg Asp Ala Tyr Tyr Gly Phe Tyr Ala Met Asp Tyr Trp
```

115 120 125

Gly Gln Gly Thr Ser Val Thr Val Ser Ser

130 135

INFORMATION FOR SEQ ID NO:9

SEQUENCE CHARACTERISTICS:

LENGTH: 15

TYPE: amino acid

TOPOLOGY: linear

MOLECULE TYPE: protein

ORIGINAL SOURCE:

CELL LINE: 1.4

SEQUENCE DESCRIPTION: SEQ ID NO:9:

Arg Ala Ser Glu Ser Val Asp Ser Tyr Gly Ile Ser Phe Met His

1 5 10 15

INFORMATION FOR SEQ ID NO:10

SEQUENCE CHARACTERISTICS:

LENGTH: 7

TYPE: amino acid

TOPOLOGY: linear

MOLECULE TYPE: protein

ORIGINAL SOURCE:

CELL LINE: 1.4

SEQUENCE DESCRIPTION: SEQ ID NO:10:

Arg Ala Ser Asn Leu Glu Ser
1 5

INFORMATION FOR SEQ ID NO:11

SEQUENCE CHARACTERISTICS:

LENGTH: 9

TYPE: amino acid

TOPOLOGY: linear

MOLECULE TYPE: protein

ORIGINAL SOURCE:

CELL LINE: 1.4

SEQUENCE DESCRIPTION: SEQ ID NO:11:

Gln Gln Ser Asn Glu Asp Pro Phe Thr
1 5

INFORMATION FOR SEQ ID NO:12

SEQUENCE CHARACTERISTICS:

LENGTH: 6

TYPE: amino acid

TOPOLOGY: linear

MOLECULE TYPE: protein

ORIGINAL SOURCE:

CELL LINE: 1.4

SEQUENCE DESCRIPTION: SEQ ID NO:12:

Ser Asp Tyr Ala Trp Asn
1 5

INFORMATION FOR SEQ ID NO:13

SEQUENCE CHARACTERISTICS:

LENGTH: 16

TYPE: amino acid

TOPOLOGY: linear

MOLECULE TYPE: protein

ORIGINAL SOURCE:

CELL LINE: 1.4

SEQUENCE DESCRIPTION: SEQ ID NO:13:

Tyr Ile Arg Tyr Ser Gly Tyr Thr Ser Tyr Asn Pro Ser Leu Lys Ser
1 5 10 15

INFORMATION FOR SEQ ID NO:14

SEQUENCE CHARACTERISTICS:

LENGTH: 9

TYPE: amino acid

TOPOLOGY: linear

MOLECULE TYPE: protein

ORIGINAL SOURCE:

CELL LINE: 1.4

SEQUENCE DESCRIPTION: SEQ ID NO:14:

Asp Leu Asp Ala Trp Tyr Phe Asp Val
1 5

INFORMATION FOR SEQ ID NO:15

SEQUENCE CHARACTERISTICS:

LENGTH: 11

TYPE: amino acid

TOPOLOGY: linear

MOLECULE TYPE: protein

ORIGINAL SOURCE:

CELL LINE: 10.1

SEQUENCE DESCRIPTION: SEQ ID NO:15:

Lys Ala Ser Glu Asn Val Val Thr Tyr Val Ser
1 5 10

INFORMATION FOR SEQ ID NO:16

SEQUENCE CHARACTERISTICS:

LENGTH: 7

TYPE: amino acid

TOPOLOGY: linear

MOLECULE TYPE: protein

ORIGINAL SOURCE:

CELL LINE: 10.1

SEQUENCE DESCRIPTION: SEQ ID NO:16:

Gly Ala Ser Asn Arg Tyr Thr
1 5

INFORMATION FOR SEQ ID NO:17

SEQUENCE CHARACTERISTICS:

LENGTH: 9

TYPE: amino acid

TOPOLOGY: linear

MOLECULE TYPE: protein

ORIGINAL SOURCE:

CELL LINE: 10.1

SEQUENCE DESCRIPTION: SEQ ID NO:17:

Gly Gln Gly Tyr Ser Tyr Pro Tyr Thr
1 5

INFORMATION FOR SEQ ID NO:18

SEQUENCE CHARACTERISTICS:

LENGTH: 5

TYPE: amino acid

TOPOLOGY: linear

MOLECULE TYPE: protein

ORIGINAL SOURCE:

CELL LINE: 10.1

SEQUENCE DESCRIPTION: SEQ ID NO:18:

Asp Phe Gly Val Asn
1 5

INFORMATION FOR SEQ ID NO:19

SEQUENCE CHARACTERISTICS:

LENGTH: 16

TYPE: amino acid

TOPOLOGY: linear

MOLECULE TYPE: protein

ORIGINAL SOURCE:

CELL LINE: 10.1

SEQUENCE DESCRIPTION: SEQ ID NO:19:

Met Ile Trp Thr Asp Gly Ile Thr Asp Tyr Asn Ser Val Leu Lys Ser
1 5 10 15

INFORMATION FOR SEQ ID NO:20

SEQUENCE CHARACTERISTICS:

LENGTH: 11

TYPE: amino acid

TOPOLOGY: linear

MOLECULE TYPE: protein

ORIGINAL SOURCE:

CELL LINE: 10.1

SEQUENCE DESCRIPTION: SEQ ID NO:20:

Asp Ala Tyr Tyr Gly Phe Tyr Ala Met Asp Tyr
1 5 10

INFORMATION FOR SEQ ID NO:21

SEQUENCE CHARACTERISTICS:

LENGTH: 22

TYPE: nucleic acid

TOPOLOGY: linear

MOLECULE TYPE: other nucleic acid (synthetic)

SEQUENCE DESCRIPTION: SEQ ID NO:21:

GGCACCTCCA GATGTTAACT GC 22

INFORMATION FOR SEQ ID NO:22

SEQUENCE CHARACTERISTICS:

LENGTH: 20

TYPE: nucleic acid

TOPOLOGY: linear

MOLECULE TYPE: other nucleic acid (synthetic)

SEQUENCE DESCRIPTION: SEQ ID NO:22:

GAARTAVCCC TTGACCAGGC 20

INFORMATION FOR SEQ ID NO:23

SEQUENCE CHARACTERISTICS:

LENGTH: 48

TYPE: nucleic acid

TOPOLOGY: linear

MOLECULE TYPE: other nucleic acid (synthetic)

FEATURE:

LOCATION: 36,37,41,42,46,47

OTHER INFORMATION: N is inosine (I).

SEQUENCE DESCRIPTION: SEQ ID NO:23:

CUACUACUAC UAGGCCACGC GTCGACTAGT ACGGGNNGGG NNGGGNNG 48

INFORMATION FOR SEQ ID NO:24

SEQUENCE CHARACTERISTICS:

LENGTH: 46

TYPE: nucleic acid

TOPOLOGY: linear

MOLECULE TYPE: other nucleic acid (synthetic)

SEQUENCE DESCRIPTION: SEQ ID NO:24:

TATAGAGCTC AAGCTTGGAT GGTGGGAAGA TGGATACAGT TGGTGC 46

INFORMATION FOR SEQ ID NO:25

SEQUENCE CHARACTERISTICS:

LENGTH: 50

TYPE: nucleic acid

TOPOLOGY: linear

MOLECULE TYPE: other nucleic acid (synthetic)

SEQUENCE DESCRIPTION: SEQ ID NO:25:

TATAGAGCTC AAGCTTCCAG TGGATAGACH GATGGGGSTG TYGTTTTGGC 50

INFORMATION FOR SEQ ID NO:26

SEQUENCE CHARACTERISTICS:

LENGTH: 20

TYPE: nucleic acid

TOPOLOGY: linear

MOLECULE TYPE: other nucleic acid (synthetic)

SEQUENCE DESCRIPTION: SEQ ID NO:26:

ACCGAGCTCG GATCCACTAG 20

INFORMATION FOR SEQ ID NO:27

SEQUENCE CHARACTERISTICS:

LENGTH: 21

TYPE: nucleic acid

TOPOLOGY: linear

MOLECULE TYPE: other nucleic acid (synthetic)

SEQUENCE DESCRIPTION: SEQ ID NO:27:

ATGCATGCTC GAGCGGCCGC C 21

## Claims

1. A monoclonal antibody capable of inhibiting the activity of human type II phospholipase A2 as well as that of type II phospholipase A2 derived from monkey and/or mouse, or a protein comprising a part thereof having said inhibitory activity.

2. A monoclonal antibody capable of releasing type II phospholipase A2 bound to the cellular membrane, or a protein comprising a part thereof having said activity.

3. The monoclonal antibody or protein according to claim 2 wherein said phospholipase A2 is derived from human.

4. A monoclonal antibody capable of not only inhibiting the activity of human type II phospholipase A2 as well as that of type II phospholipase A2 derived from monkey and/or mouse, but also releasing type II phospholipase A2 bound to the cell membrane or a protein comprising a part thereof having said activity.

5. A monoclonal antibody produced by any one of hybridomas 12H5 (FERM BP-5300), 1.4 (FERM BP-5297) and 10.1 (FERM BP-5298) or a protein comprising a part thereof, or a monoclonal antibody or a protein comprising a part thereof having the activity against type II phospholipase A2 equivalent to that of said monoclonal antibody or protein.

6. The monoclonal antibody or protein comprising a part thereof according to claim 1 or 2, wherein said monoclonal antibody or protein comprises protein having the amino acid sequence represented by any one of SEQ ID NO:2, 4, 6 or 8 or those modified by substitution, deletion or insertion of one or more amino acid residues contained in said sequence.

7. The monoclonal antibody or protein comprising a part thereof according to claim 1 or 2, wherein said monoclonal antibody or protein comprises protein having the amino acid sequence represented by any one of SEQ ID NO:9 to 20 or those modified by substitution, deletion or insertion of one or more amino acid residues contained in said sequence.

8. A cell producing the monoclonal antibody or protein according to any one of claims 1 to 7.

9. The cell according to claim 8 wherein said cell is a hybridoma.

10. The cell according to claim 8 wherein said cell is a cell transformed with recombinant DNA.

11. A method for producing the monoclonal antibody or protein according to any one of claims 1 to 7, wherein said method comprises processes of culturing the cell according to claim 8 and recovering monoclonal antibodies or proteins from the supernatant of growth medium.

12. A DNA encoding the monoclonal antibody or protein according to any one of claims 1 to 7.

13. The DNA according to claim 12, wherein said DNA comprises the base sequence with any one of SEQ ID NO:1, 3, 5 or 7, or that modified by substitution, deletion or insertion of one or more amino acid residues contained in said sequence.

14. A recombinant vector comprising the DNA according to claim 12 or 13.

15. A medical composition comprising the monoclonal antibody or protein according to any one of claims 1 to 7 and a pharmacologically acceptable carrier.

16. A type II phospholipase A2 inhibitor comprising the monoclonal antibody or protein according to any one of claims 1 to 7.

Figure 1

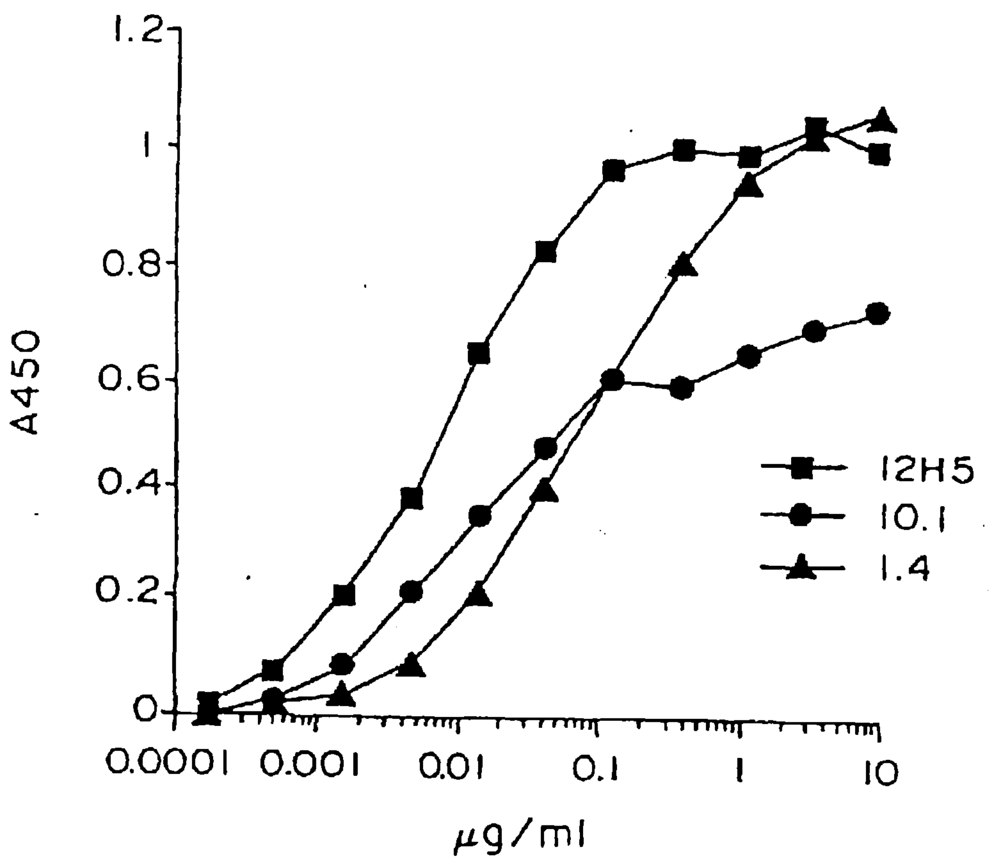

Figure 2

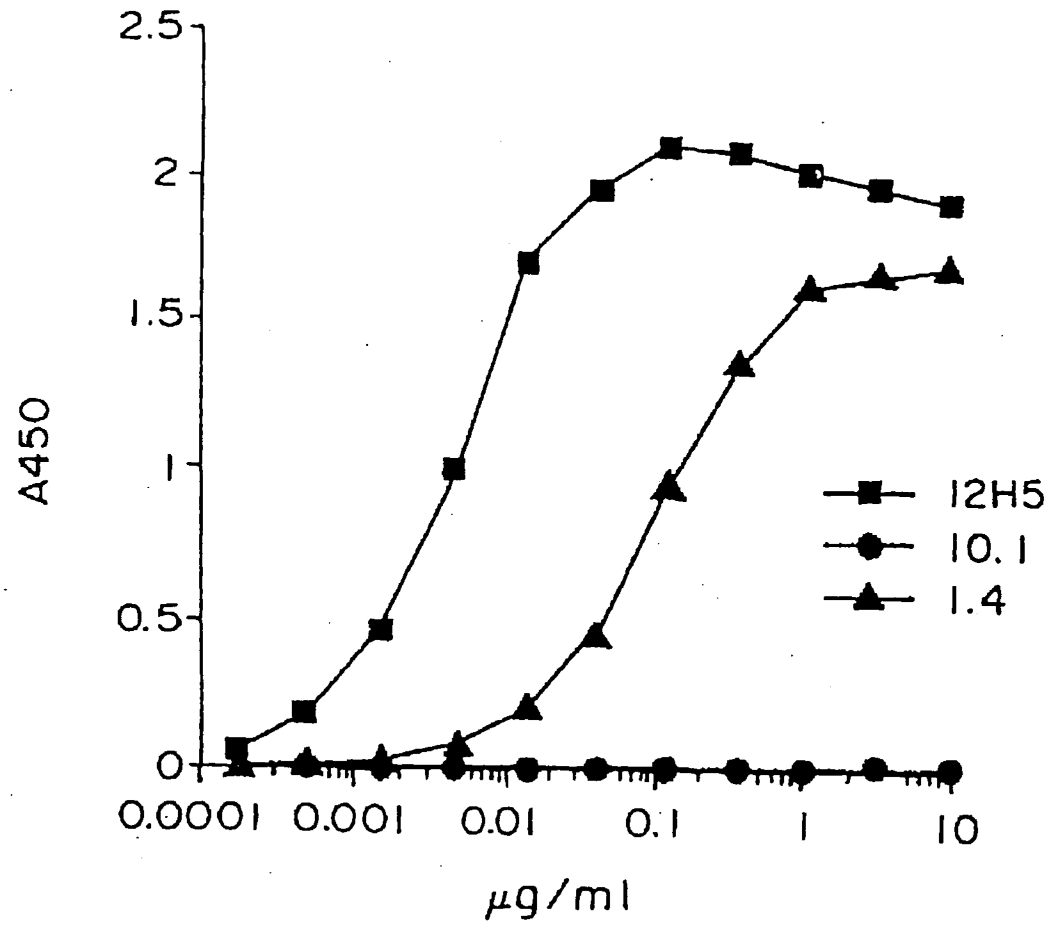

Figure 3

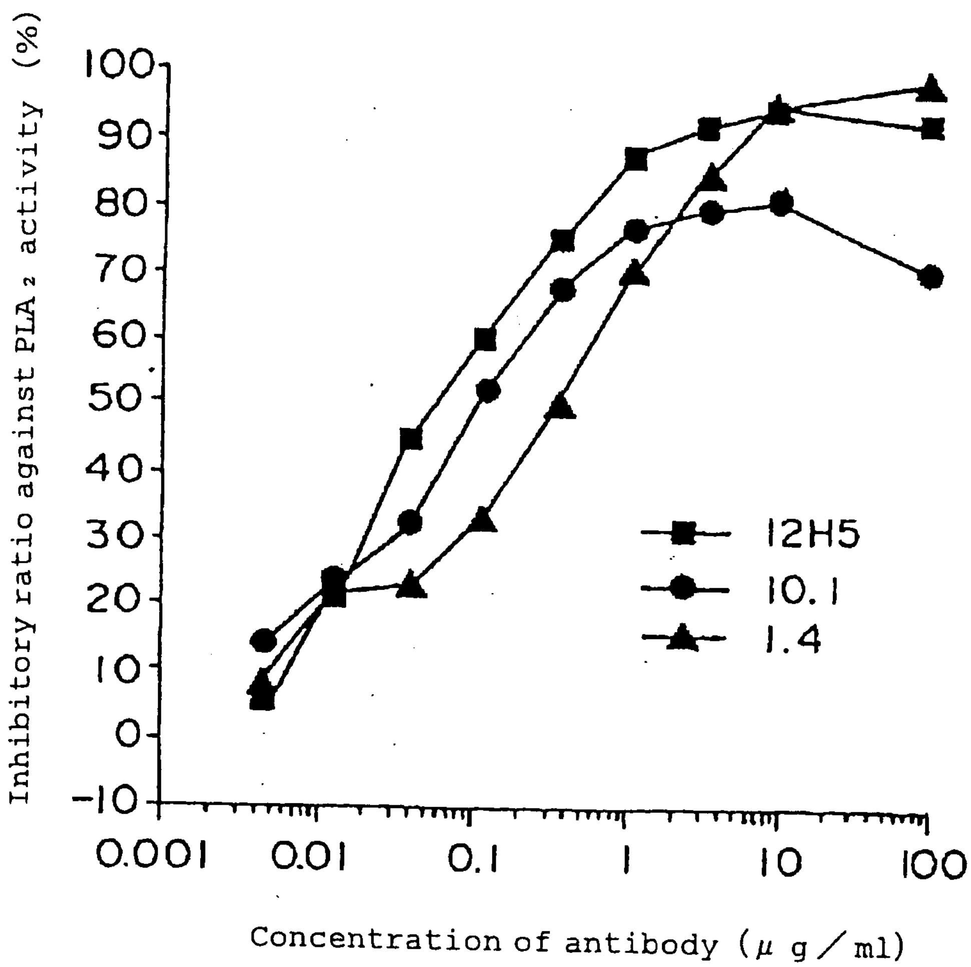
Inhibitory ratio against PLA₂ activity (%)
100
90
80
70
60
50
40
30
20
10
0
-10
0.001
0.01
0.1
1
10
100
12H5
10.1
1.4
Concentration of antibody (μg／ml)

Figure 4

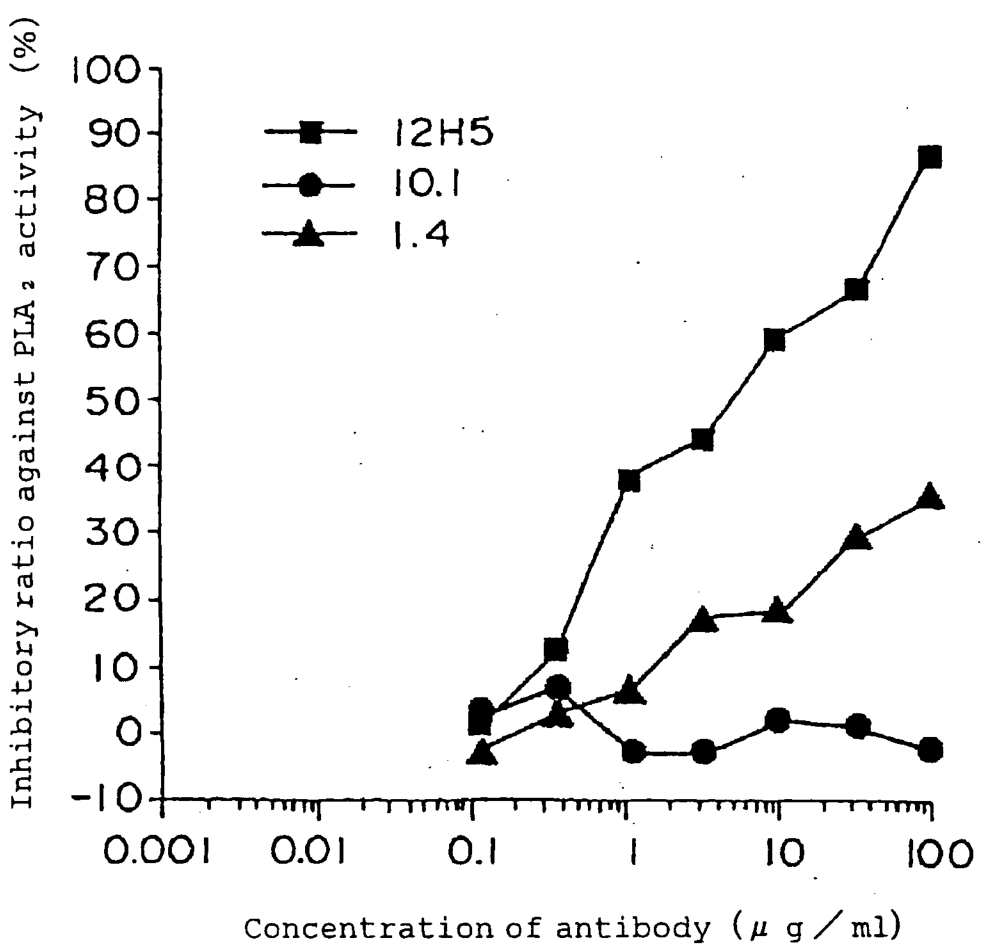
Inhibitory ratio against PLA₂ activity (%)
100
90
80
70
60
50
40
30
20
10
0
-10
12H5
10.1
1.4
0.001
0.01
0.1
1
10
100
Concentration of antibody (μg/ml)

Figure 5

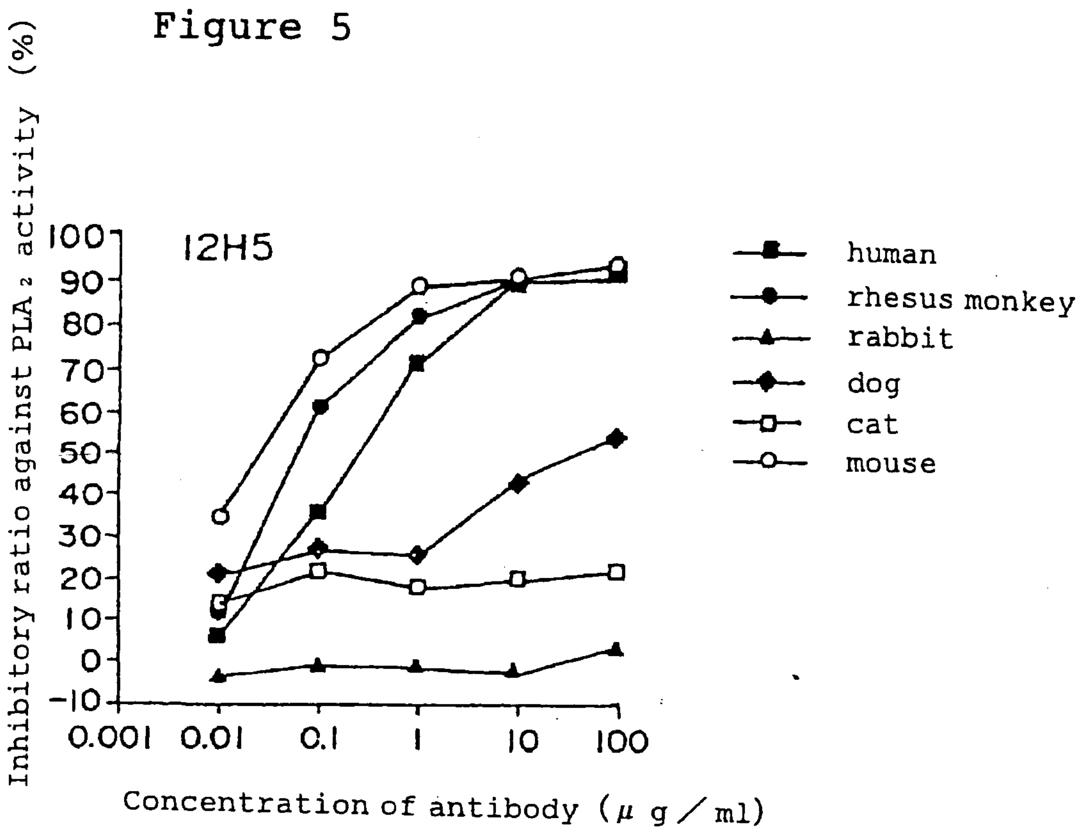
Inhibitory ratio against PLA₂ activity (%)
100
90
80
70
60
50
40
30
20
10
0
-10
12H5
0.001
0.01
0.1
1
10
100
Concentration of antibody (μ g / ml)
human
rhesus monkey
rabbit
dog
cat
mouse

Figure 6

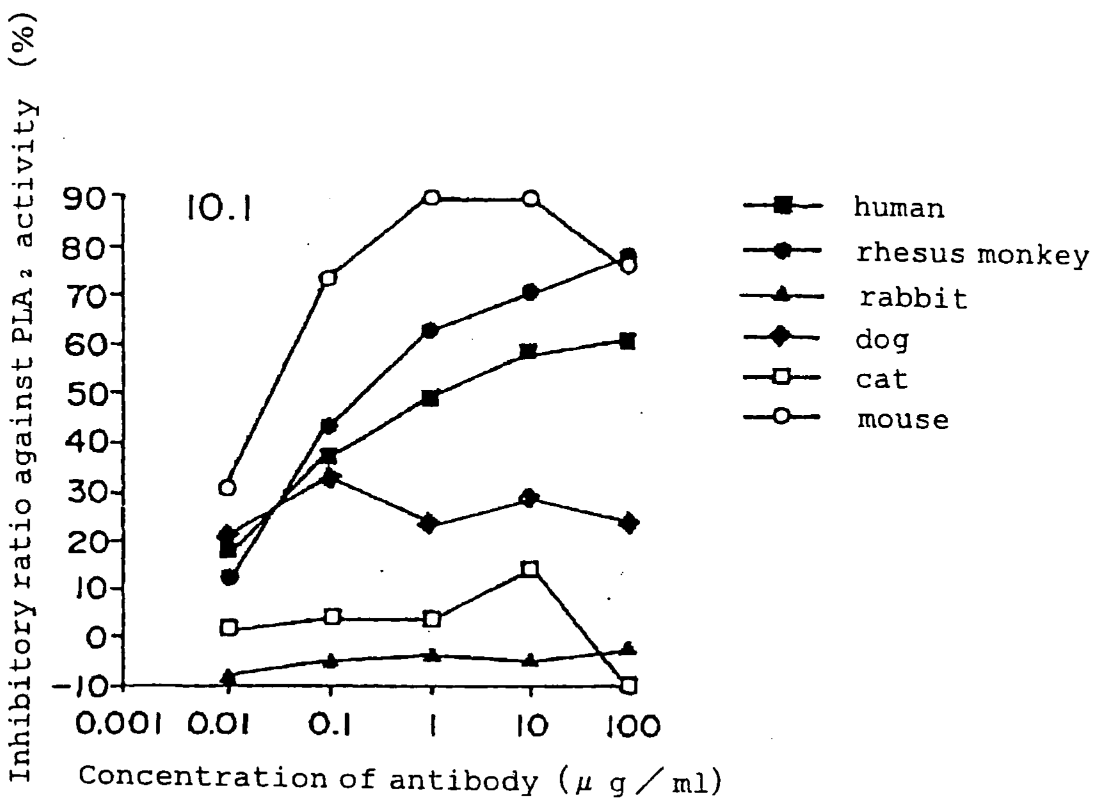
Inhibitory ratio against PLA₂ activity (%)
90
80
70
60
50
40
30
20
10
0
−10
10.1
0.001
0.01
0.1
1
10
100
Concentration of antibody (μg／ml)
human
rhesus monkey
rabbit
dog
cat
mouse

Figure 7

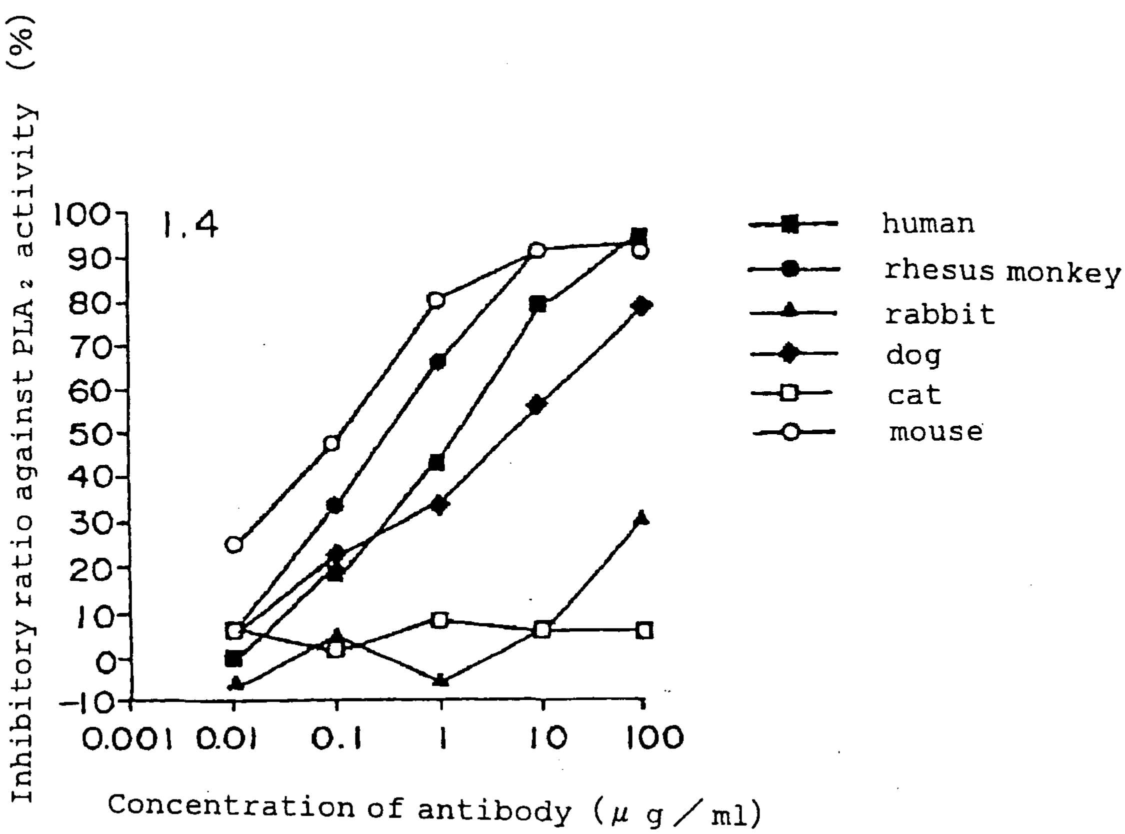
Inhibitory ratio against PLA₂ activity (%)
100
90
80
70
60
50
40
30
20
10
0
-10
1.4
0.001
0.01
0.1
1
10
100
Concentration of antibody (μg/ml)
human
rhesus monkey
rabbit
dog
cat
mouse

Figure 8

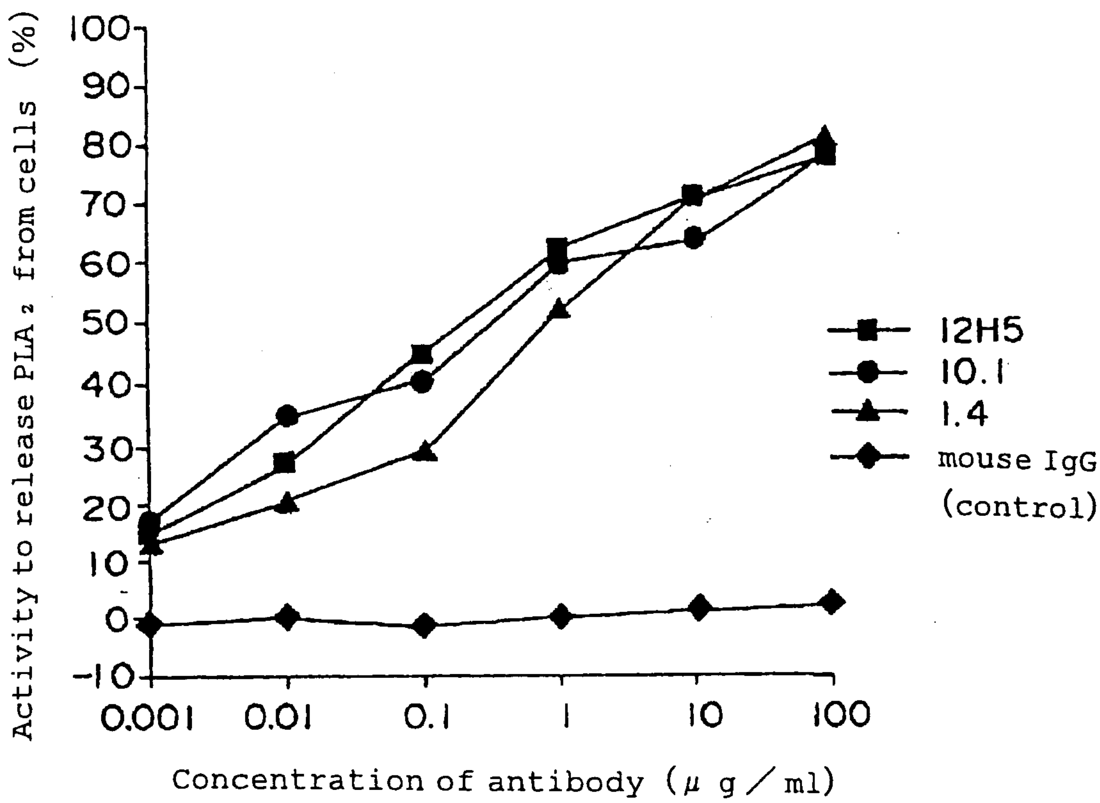
Activity to release PLA₂ from cells (%)
100
90
80
70
60
50
40
30
20
10
0
-10
0.001
0.01
0.1
1
10
100
Concentration of antibody (μg／ml)
12H5
10.1
1.4
mouse IgG
(control)

Figure 9

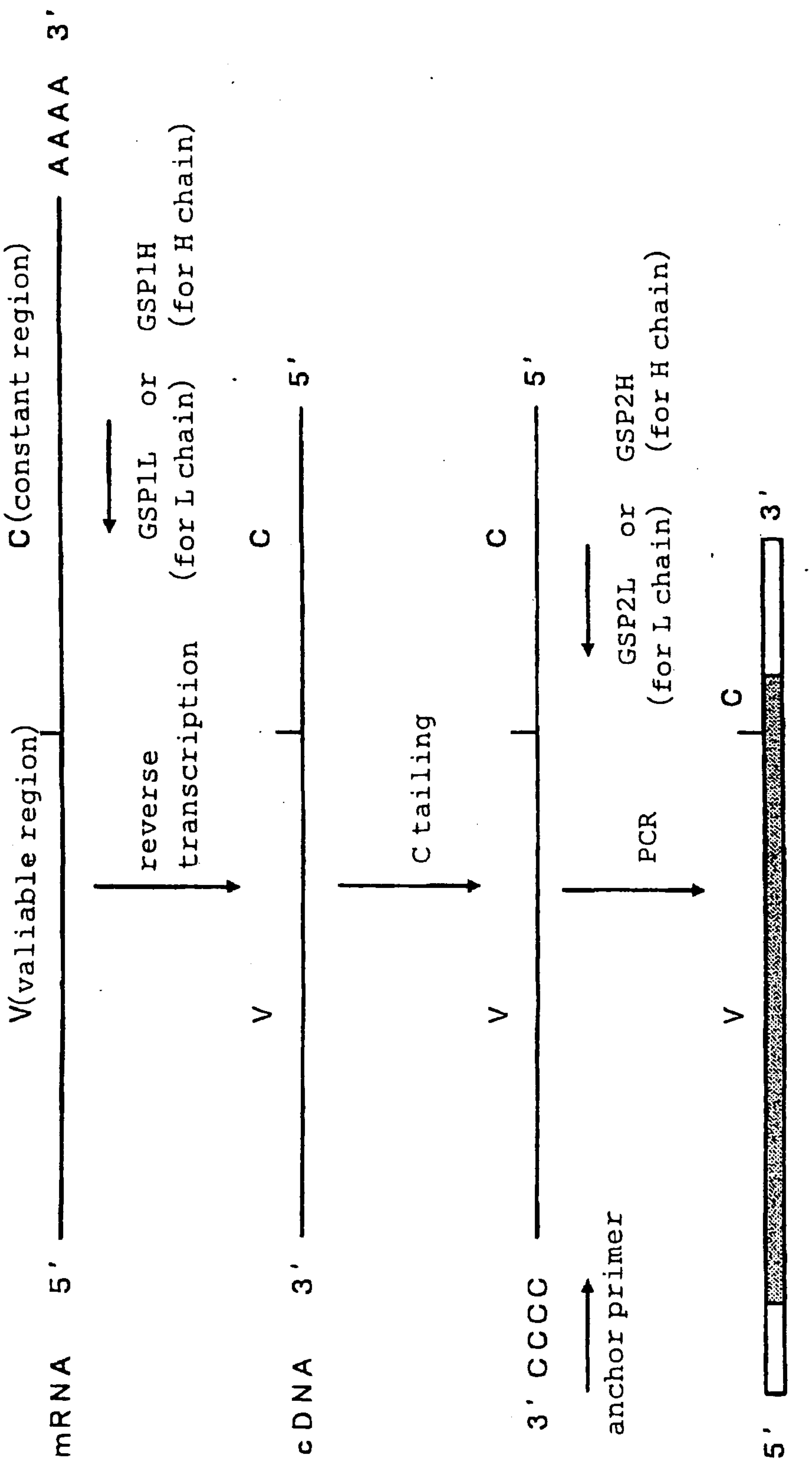
mRNA
5'
V(valiable region)
C(constant region)
AAAA
3'
GSP1L or GSP1H
(for L chain) (for H chain)
reverse
transcription
cDNA
3'
V
C
5'
C tailing
3' CCCC
V
C
5'
anchor primer
GSP2L or GSP2H
(for L chain) (for H chain)
PCR
5'
V
C
3'

Figure 10

ATGGAGACAGACACACTCCTGCTATGGGTGCTGCTGCTCTGGGTTCCAGGTTCCACAGGT 60
MetGluThrAspThrLeuLeuLeuTrpValLeuLeuLeuTrpValProGlySerThrGly

GACATTGTGCTGACCCAATCTCCAGCTTCCTTGGCTGTGTCTTTAGGGCAGAGGGCCACC 120
AspIleValLeuThrGlnSerProAlaSerLeuAlaValSerLeuGlyGlnArgAlaThr

ATATCCTGCAGAGCCAGTGAAAGTGTTGATAGTTATGGCATTAGTTTTATGCACTGGTAT 180
IleSerCysArgAlaSerGluSerValAspSerTyrGlyIleSerPheMetHisTrpTyr

CAGCAGAAACCAGGACAGCCCCCCAAACTCCTCATTTATCGTGCATCCAACCTAGAATCT 240
GlnGlnLysProGlyGlnProProLysLeuLeuIleTyrArgAlaSerAsnLeuGluSer

GGGATCCCTGCCAGGTTTAGTGGCAGTGGGTCTAGGACAGAATTCACCCTCACCATTAAT 300
GlyIleProAlaArgPheSerGlySerGlySerArgThrGluPheThrLeuThrIleAsn

CCTGTGGAGGCTGATGATGTTGCAACCTATCACTGTCAGCAAAGTAATGAGGATCCATTC 360
ProValGluAlaAspAspValAlaThrTyrHisCysGlnGlnSerAsnGluAspProPhe

ACGTTCGGCTCGGGGACAAAGTTGGAAATAAAA 393
ThrPheGlySerGlyThrLysLeuGluIleLys

Figure 11

ATGAGAGTGCTGATTCTTTTGTGGCTGTTCACAGCCTTTCCTGGTTTCCTGTCTGATGTG 60
MetArgValLeuIleLeuLeuTrpLeuPheThrAlaPheProGlyPheLeuSerAspVal

CAGCTTCAGGAATCGGGACCTGGCCTGGTGAAACCTTCTCAATCTCTGTCCCTCACCTGC 120
GlnLeuGlnGluSerGlyProGlyLeuValLysProSerGlnSerLeuSerLeuThrCys

ATGGTCACTGGCTACTCAATCACCAGTGATTATGCCTGGAACTGGATCCGGCAGTTTCCG 180
MetValThrGlyTyrSerIleThrSerAspTyrAlaTrpAsnTrpIleArgGlnPhePro

GGAAACAAACTGGAGCGGATGGGATACATAAGGTACAGTGGTTACACTAGCTACAACCCA 240
GlyAsnLysLeuGluArgMetGlyTyrIleArgTyrSerGlyTyrThrSerTyrAsnPro

TCTCTCAAAAGTCGAATCTTTATCACGCGAGACACATCCCAGAACCAGTTCTTCCTACAT 300
SerLeuLysSerArgIlePheIleThrArgAspThrSerGlnAsnGlnPhePheLeuHis

TTGACTTCTGTGACTACTGAGGACACAGCCACATATTACTGTACAAGAGACTTGGACGCC 360
LeuThrSerValThrThrGluAspThrAlaThrTyrTyrCysThrArgAspLeuAspAla

TGGTACTTCGATGTTTGGGGCGCAGGGACAACGGTCACCGTCTCCTCA 408
TrpTyrPheAspValTrpGlyAlaGlyThrThrValThrValSerSer

Figure 12

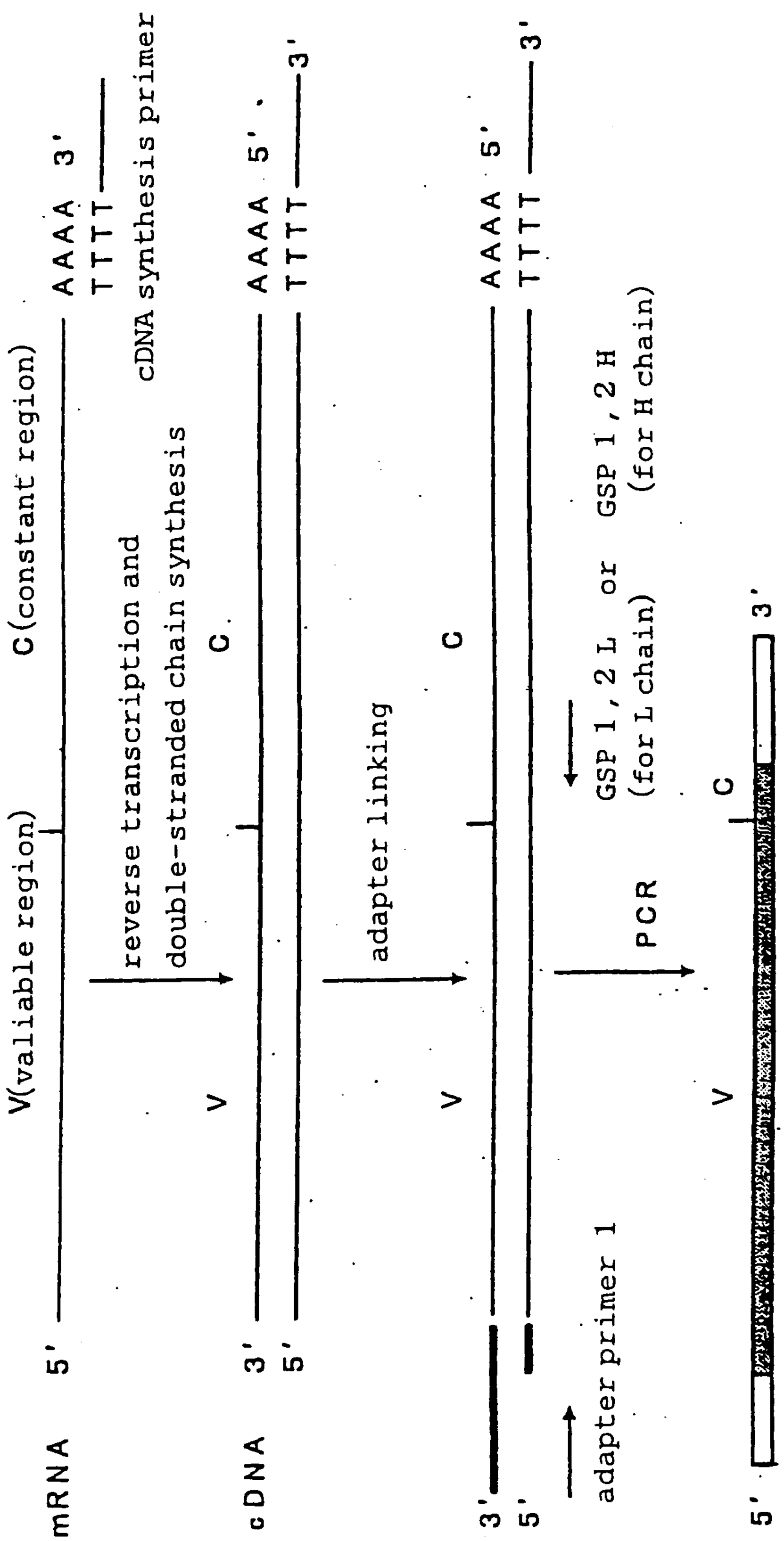
mRNA
5'
V(valiable region)
C(constant region)
AAAA 3'
TTTT
cDNA synthesis primer
reverse transcription and
double-stranded chain synthesis
cDNA
3'
5'
V
C
AAAA 5'
TTTT
3'
adapter linking
3'
5'
V
C
AAAA 5'
TTTT
3'
adapter primer 1
PCR
GSP 1, 2 L
(for L chain)
or
GSP 1, 2 H
(for H chain)
5'
V
C
3'

Figure 13

ATGGAATCACAGACTCTGGTCTTCATATCCATACTGCTCTGGTTATATGGAGCTGATGGG 60
MetGluSerGlnThrLeuValPheIleSerIleLeuLeuTrpLeuTyrGlyAlaAspGly

AACATTGTAATGACCCAATCTCCCAAATCCATGTCCATGTCAGTAGGAGAGAGGGTCACC 120
AsnIleValMetThrGlnSerProLysSerMetSerMetSerValGlyGluArgValThr

TTGACCTGCAAGGCCAGTGAGAATGTGGTTACTTATGTTTCCTGGTATCAACAGAAACCA 180
LeuThrCysLysAlaSerGluAsnValValThrTyrValSerTrpTyrGlnGlnLysPro

GAGCAGTCTCCTAAACTGCTGATATACGGGGCATCCAACCGGTACACTGGGGTCCCCGAT 240
GluGlnSerProLysLeuLeuIleTyrGlyAlaSerAsnArgTyrThrGlyValProAsp

CGCTTCACAGGCAGTGGATCTGCAACAGATTTCACTCTGACCATCAGCAGTGTGCAGGCT 300
ArgPheThrGlySerGlySerAlaThrAspPheThrLeuThrIleSerSerValGlnAla

GAAGACCTTGCAGATTATCACTGTGGACAGGGTTACAGCTATCCGTACACGTTCGGAGGG 360
GluAspLeuAlaAspTyrHisCysGlyGlnGlyTyrSerTyrProTyrThrPheGlyGly

GGGACCAAGCTGGAAATAAAA 381
GlyThrLysLeuGluIleLys

Figure 14

ATGGCTGTCCTGGCATTACTTTTTTGCCTGGTAACATTCCCAAGCTGTATCCTTTCCCAG 60
MetAlaValLeuAlaLeuLeuPheCysLeuValThrPheProSerCysIleLeuSerGln

GTGCAGCTGAAGGAGTCAGGACCTGGCCTGGTGGCGCCCTCACAGAGCCTGTCCATCACA 120
ValGlnLeuLysGluSerGlyProGlyLeuValAlaProSerGlnSerLeuSerIleThr

TGTACCGTCTCAGGGTTCTCATTAACCGACTTTGGTGTAAACTGGGTTCGCCAGCCTCCA 180
CysThrValSerGlyPheSerLeuThrAspPheGlyValAsnTrpValArgGlnProPro

GGAAAGGGTCTGGAGTGGCTGGGAATGATATGGACTGATGGAATCACAGACTATAATTCA 240
GlyLysGlyLeuGluTrpLeuGlyMetIleTrpThrAspGlyIleThrAspTyrAsnSer

GTTCTCAAATCCAGACTGAGCATCAGCAAGGACACCTCCAAGAGCCAAGTTTTCTTGAAA 300
ValLeuLysSerArgLeuSerIleSerLysAspThrSerLysSerGlnValPheLeuLys

ATGAACAATCTGCAAACTGATGACACAGCCAGGTACTACTGTGCCAGAGATGCATACTAC 360
MetAsnAsnLeuGlnThrAspAspThrAlaArgTyrTyrCysAlaArgAspAlaTyrTyr

GGCTTCTATGCTATGGACTACTGGGGTCAAGGAACCTCAGTCACCGTCTCCTCA 414
GlyPheTyrAlaMetAspTyrTrpGlyGlnGlyThrSerValThrValSerSer

## INTERNATIONAL SEARCH REPORT

International application No.
PCT/JP95/02714

**A. CLASSIFICATION OF SUBJECT MATTER**

Int. Cl$^6$ C07K16/40, C07K16/18, C12P21/08, C21N15/06, C12N15/13, C12N5/20, C12N9/99, A61K39/395// (C12P21/08, C12R1:91)

According to International Patent Classification (IPC) or to both national classification and IPC

**B. FIELDS SEARCHED**

Minimum documentation searched (classification system followed by classification symbols)

Int. Cl$^6$ C12P21/00, C12P21/02, C12P21/08, C12N15/02-15/90

Documentation searched other than minimum documentation to the extent that such documents are included in the fields searched

Electronic data base consulted during the international search (name of data base and, where practicable, search terms used)

BIOSIS PREVIEWS, WPI, WPI/L, CAS ONLINE

**C. DOCUMENTS CONSIDERED TO BE RELEVANT**

| Category* | Citation of document, with indication, where appropriate, of the relevant passages | Relevant to claim No. |
|---|---|---|
| A | JP, 63-258898, A (Shinogi & Co., Ltd.), October 26, 1988 (26. 10. 88) & EP, 287397, A & US, 4978609, A | 1 - 16 |
| A | JP, 4-36193, A (Shinogi & Co., Ltd.), February 6, 1992 (06. 02. 92) & EP, 459450, A & US, 5358849, A | 1 - 16 |
| A | JP, 7-109300, A (Teijin Ltd.), April 25, 1995 (24. 04. 95) (Family: none) | 1 - 16 |
| A | Takayama, K. et al. "Monoclonal antibodies against human synovial phosphlipase A2", Biochem. Biophys. Res. Commun. (1990) Vol. 167, No. 3, p. 1309-1315 | 1 - 16 |
| A | Storner, C.R. et al. "Human group II phospholipase A2 characterization of monoclonal antibodies and immunochemical qunatitation of the protein in synovial fluid", J. Immunol. Methods (1991) Vol. 145, Nos 1 to 2, p. 127-136 | 1 - 16 |

[X] Further documents are listed in the continuation of Box C. [ ] See patent family annex.

* Special categories of cited documents:
- "A" document defining the general state of the art which is not considered to be of particular relevance
- "E" earlier document but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| April 1, 1996 (01. 04. 96) | April 16, 1996 (16. 04. 96) |
| Name and mailing address of the ISA/ Japanese Patent Office | Authorized officer |
| Facsimile No. | Telephone No. |

Form PCT/ISA/210 (second sheet) (July 1992)

INTERNATIONAL SEARCH REPORT

International application No.
PCT/JP95/02714

C (Continuation). DOCUMENTS CONSIDERED TO BE RELEVANT

| Category* | Citation of document, with indication, where appropriate, of the relevant passages | Relevant to claim No. |
|---|---|---|
| A | Misaki, A. et al. "Production and characterization of monoclonal antibodies against human pancreatic phospholipase A2", J. Clin. Biochem. Nutr. (1991) Vol. 11, No. 2 p. 79-90 | 1 - 16 |
| A | Murakami, M. et al. "Preparation of antibodies to phospholipase A2", Methods Enzymol. (1991) Vol. 197, p. 223-233 | 1 - 16 |

Form PCT/ISA/210 (continuation of second sheet) (July 1992)